(19) Europäisches Patentamt
European Patent Office
Office européen des brevets

(11) EP 4 309 515 A1

# (12) EUROPEAN PATENT APPLICATION

published in accordance with Art. 153(4) EPC

(43) Date of publication:
**24.01.2024 Bulletin 2024/04**

(21) Application number: **22784603.7**

(22) Date of filing: **30.03.2022**

(51) International Patent Classification (IPC):
***A23L 33/10*** (2016.01) ***A23F 3/16*** (2006.01)
***A23F 5/24*** (2006.01) ***A23G 3/36*** (2006.01)
***A23G 9/32*** (2006.01) ***A23L 2/52*** (2006.01)
***A61K 9/06*** (2006.01) ***A61K 9/08*** (2006.01)
***A61K 9/107*** (2006.01) ***A61K 9/16*** (2006.01)
***A61K 9/20*** (2006.01) ***A61K 9/48*** (2006.01)
***A61K 31/05*** (2006.01) ***A61K 31/122*** (2006.01)
***A61K 31/7048*** (2006.01) ***A61P 1/04*** (2006.01)
***A61P 3/04*** (2006.01) ***A61P 3/10*** (2006.01)
***A61P 43/00*** (2006.01) ***C12G 3/05*** (2019.01)

(52) Cooperative Patent Classification (CPC):
**A23F 3/16; A23F 5/24; A23G 3/36; A23G 9/32; A23L 2/52; A23L 33/10; A61K 9/06; A61K 9/08; A61K 9/107; A61K 9/16; A61K 9/20; A61K 9/48; A61K 31/05; A61K 31/122; A61K 31/7048;** (Cont.)

(86) International application number:
**PCT/JP2022/015872**

(87) International publication number:
**WO 2022/215619 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.04.2021 JP 2021065419**

(71) Applicant: **Hayashibara Co., Ltd.**
**Okayama-shi, Okayama 702-8006 (JP)**

(72) Inventors:
- **TAKAHASHI, Jun**
  **Okayama-shi Okayama 702-8006 (JP)**
- **YAMADA, Mika**
  **Okayama-shi Okayama 702-8006 (JP)**
- **ARAI, Norie**
  **Okayama-shi Okayama 702-8006 (JP)**
- **HASHINI, Emiko**
  **Okayama-shi Okayama 702-8006 (JP)**
- **MITSUZUMI, Hitoshi**
  **Okayama-shi Okayama 702-8006 (JP)**

(74) Representative: **Page White Farrer**
**Bedford House**
**21a John Street**
**London WC1N 2BF (GB)**

## (54) COMPOSITION FOR PROMOTING GLP-1 SECRETION

(57) An object of the present invention is to provide a composition having an effective GLP-1 secretion-promoting effect. The present invention attains this object by providing a composition for promoting GLP-1 secretion, comprising component (A) and component (B) as active ingredients, wherein said component (A) is a glycosyl naringenin and said component (B) is one or more selected from a group consisting of α-pinene, β-pinene, sabinene, camphene, valencene, β-caryophyllene, β-ionone, methyl anthranilate, methyl N-methylanthranilate, thymol, and nootkatone.

EP 4 309 515 A1

(52) Cooperative Patent Classification (CPC): (Cont.)
**A61P 1/04; A61P 3/04; A61P 3/10; A61P 43/00; C12G 3/05**

## Description

### Technical Field

The present invention relates to a composition for promoting GLP-1 secretion. In more detail, the present invention relates to a composition for promoting GLP-1 secretion, comprising component (A) and component (B) as active ingredients, wherein component (A) is a glycosyl naringenin and component (B) is one or more selected from a group consisting of α-pinene, β-pinene, sabinene, camphene, valencene, β-caryophyllene, β-ionone, methyl anthranilate, methyl N-methylanthranilate, thymol, and nootkatone.

### Background Art

It is known that endocrine cells are scattered throughout the intestinal epithelium, and gastrointestinal hormones secreted from these intestinal endocrine cells regulate functions of other organs. A part of the gastrointestinal hormones secreted from endocrine cells is also called incretins, which are secreted into the blood with meal intake and act on β-cells in the islets of Langerhans (hereinafter referred to as "pancreatic β-cells") in the pancreas, promoting insulin secretion.

Examples of incretins include glucose-dependent insulinotropic polypeptide (hereinafter referred to as "GIP") and glucagon-like peptide-1 (hereinafter referred to as "GLP-1"). GIP is secreted from K cells present in the upper small intestine. Meanwhile, GLP-1 is secreted from L cells present in the lower small intestine.

GLP-1 is secreted into the blood with meal intake. It acts on pancreatic β-cells, promotes insulin secretion, and thereby lowers blood glucose levels. It has also been shown that GLP-1 inhibits glucagon secretion from pancreatic α-cells, suppresses appetite and food intake in the central nervous system, and delays gastric excretion (Non-Patent Literature 1). Furthermore, since GLP-1 receptors are expressed not only in pancreatic β-cells but also in many organs and tissues in the body, such as the kidney, GLP-1 is thought to have various physiological effects other than those described above. For example, GLP-1 has been shown to have the effect of lowering the blood pressure based on human studies and animal studies using rats. In addition, it has been found that GLP-1 receptor agonists promote urinary sodium excretion and suppress elevation of the blood pressure caused by angiotensin II (Non-Patent Literature 2). Furthermore, GLP-1 is also thought to play a role in suppressing arteriosclerosis, maintaining bone strength, regulating peripheral circadian rhythm, etc. (Non-Patent Literatures 3, 4 and 5). Therefore, promoting secretion of GLP-1 is thought to lead to not only maintenance of glucose tolerance, homeostasis of glucose metabolism, and improvement of obesity, but also prevention and improvement of lifestyle-related diseases as well as various homeostasis in the living body.

However, because GLP-1 is a polypeptide, it is easily digested and degraded in the gastrointestinal tract when ingested orally. Accordingly, it is thought that the absorption rate into the blood is low and the bioavailability is very low, when ingested orally. Meanwhile, it is also known that, even when administered subcutaneously or intravenously, GLP-1 is rapidly degraded by a degrading enzyme (dipeptidyl peptidase 4). Therefore, in order to increase GLP-1 concentration in the living body over a long period of time, it is rather desirable to continuously promote the secretion of endogenous GLP-1 using a method with less burden on the body, than administrating GLP-1 from outside the body.

There have been several reports on the GLP-1 secretion-promoting effect of plant extracts and their components (Patent Literatures 1 and 2). For example, Patent Literature 1 describes honeysuckles, alkanets or processed products thereof have the GLP-1 secretion-promoting effect. Meanwhile, Patent Literature 2 describes bitter melon-derived components have the GLP-1 secretion-promoting effect. The GLP-1 secretion-promoting effect of geraniol and citronellal has been also reported (Non-Patent Literature 6).

As described above, active ingredients for purpose of promoting GLP-1 secretion have been proposed. However, the conventional active ingredients having the GLP-1 secretion-promoting effect have poor water solubility and therefore could not be widely used in the field of foods. In addition, when such conventional active ingredients with poor water solubility were used, the GLP-1 secretion-promoting effect obtained was not necessarily sufficient. In the context of these backgrounds, an agent for promoting GLP-1 secretion that is more effective and can be formulated into various products has been intensely desired.

### Citation List

#### Patent Literature

[Patent Literature 1] Japanese Patent Application Publication No. 2016-138070
[Patent Literature 2] International Publication No. WO2017/159725

**Non-Patent Literature**

[Non-Patent Literature 1] Gekkan Tonyobyo (DIABETES), Supplement Volume, "Incretin", 2009/12
[Non-Patent Literature 2] Biochemical and Biophysical Research Communications 380, 44-49 (2009)
[Non-Patent Literature 3] PLoS ONE 8(8), e70933 (2013)
[Non-Patent Literature 4] Journal of Endocrinology 219, 59-68 (2013)
[Non-Patent Literature 5] PLoS ONE 8(11), e81119 (2013)
[Non-Patent Literature 6] Scientific Reports 7(1), 1-11 (2017)

**Disclosure of Invention**

**Technical Problem**

In view of the above-mentioned backgrounds, an object of the present invention is to provide a composition having a more effective GLP-1 secretion-promoting effect.

**Solution to Problem**

The present inventors accumulated research efforts in order to attain the above object and found that a composition comprising glycosyl naringenin (component (A)) together with one or more selected from a group consisting of α-pinene, β-pinene, sabinene, camphene, valencene, β-caryophyllene, β-ionone, methyl anthranilate, methyl N-methylanthranilate, thymol, and nootkatone (component (B)) promotes GLP-1 secretion, and further that a composition comprising glycosyl naringenin together with thymol and/or nootkatone at a specific ratio is particularly effective in promoting GLP-1 secretion.

In other words, the present invention attains the above object by providing the followings:

(1) A composition for promoting GLP-1 secretion, comprising component (A) and component (B) as active ingredients, wherein said component (A) is a glycosyl naringenin and said component (B) is one or more selected from a group consisting of α-pinene, β-pinene, sabinene, camphene, valencene, β-caryophyllene, β-ionone, methyl anthranilate, methyl N-methylanthranilate, thymol, and nootkatone;
(2) The composition for promoting GLP-1 secretion according to (1) above, wherein said component (A) is 3"-α-monoglucosyl naringin;
(3) The composition for promoting GLP-1 secretion according to (1) or (2) above, wherein said component (B) is thymol and/or nootkatone;
(4) The composition for promoting GLP-1 secretion according to any one of (1) to (3) above, comprising said component (A) and said component (B) at a molar ratio of 10,000:1 to 1:10;
(5) The composition for promoting GLP-1 secretion according to any one of (1) to (4) above, comprising said component (A) in an amount of 0.0001% by mass or more;
(6) The composition for promoting GLP-1 secretion according to any one of (1) to (5) above, which is for use in at least one of the followings: prevention or improvement of obesity, diabetes, and postprandial hyperglycemia; suppression of elevation of blood glucose level; inhibition of glucagon secretion; inhibition of gastric excretion and gastric acid secretion; suppression of appetite; suppression of food intake; and induction of satiety;
(7) A composition, comprising component (A) and component (B), wherein said component (A) is a glycosyl naringenin and said component (B) is one or more selected from a group consisting of α-pinene, β-pinene, sabinene, camphene, valencene, β-caryophyllene, β-ionone, methyl anthranilate, methyl N-methylanthranilate, thymol, and nootkatone;
(8) The composition according to (7) above, comprising said component (A) and said component (B) at a molar ratio of 10,000:1 to 1:10;
(9) The composition according to (7) or (8) above, wherein said component (A) is 3"-α-monoglucosyl naringin;
(10) The composition according to any one of (7) to (9) above, wherein said component (B) is thymol and/or nootkatone;
(11) The composition according to any one of (7) to (10) above, comprising said component (A) in an amount of 0.0001% by mass or more;
(12) A food or beverage, comprising the composition according to any one of (1) to (11) above;
(13) A supplement, comprising the composition according to any one of (1) to (11) above;
(14) A flavoring agent, comprising the composition according to any one of (1) to (11) above;

(15) A pharmaceutical or quasi-drug, comprising the composition according to any one of (1) to (11) above;
(16) A feed, comprising the composition according to any one of (1) to (11) above;
(17) The food or beverage according to (12) above, which is in the form of liquid, fluid, gel, semi-solid, solid or powder;
(18) The supplement according to (13) above, which is in the form of liquid, gel, paste, tablet, round tablet, capsule, powder, granule, fine granule, or troche;
(19) The flavoring agent according to (14) above, which is in the form of a water-soluble flavoring, oil-soluble flavoring, emulsified flavoring, or powdered flavoring;
(20) The pharmaceutical or quasi-drug according to (15) above, which is in the form of liquid, fluid, gel, semi-solid, solid, tablet, round tablet, capsule, powder, granule, fine granule or troche;
(21) The feed according to (16) above, which is in the form of liquid, powder, pellet, flake, or mash.

**Effects of Invention**

The composition for promoting GLP-1 secretion according to the present invention effectively promotes GLP-1 secretion.

**Description of Embodiments**

The present invention relates to a composition for promoting GLP-1 secretion, comprising component (A) and component (B) as active ingredients, wherein component (A) is a glycosyl naringenin and component (B) is one or more selected from a group consisting of α-pinene, β-pinene, sabinene, camphene, valencene, β-caryophyllene, β-ionone, methyl anthranilate, methyl N-methylanthranilate, thymol, and nootkatone. Below, component (A) and component (B), which are the active ingredients of the composition of the present invention, will be explained in the order.

The term "glycosyl naringenin" as referred to in the present description refers to a type of flavanone and is a generic term of glycosides comprising "naringenin" as an aglycone. Naringenin has a structure represented by Chemical Formula 1 shown below.

Chemical Formula 1:

[Chem. 1]

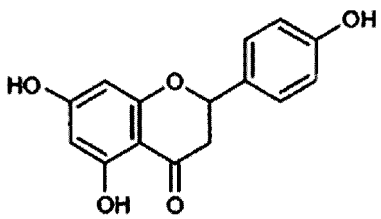

Representative examples of compounds that fall within the scope of "glycosyl naringenin" as referred to in the present description include "naringin" (Chemical Formula 2 shown below), which has a structure composed of a naringenin and a neohesperidose (α-rhamnosyl (1→2) glucose) conjugated to the C(7) hydroxyl group of the naringenin by β-glycosidic linkage; and "3"-α-monoglucosyl naringin" (Chemical Formula 3 shown below), which has a structure composed of a naringin and a glucose conjugated to the C(3) hydroxyl group of the glucose residue of the neohesperidose of the naringin (the C(3") hydroxyl group of the naringin) by α-glycosidic linkage. Naringin is contained, for example, in the unripe peel of citrus fruits and is a substance having an antioxidant effect. It has been found that naringin has the activity to induce TNF, which maintains homeostasis of the living body, as well as the physiological effects of strengthening capillaries, preventing bleeding, regulating blood pressure, lowering cholesterol, etc.

Chemical Formula 2:

[Chem. 2]

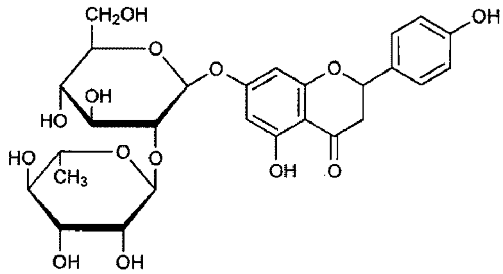

Chemical Formula 3:

[Chem. 3]

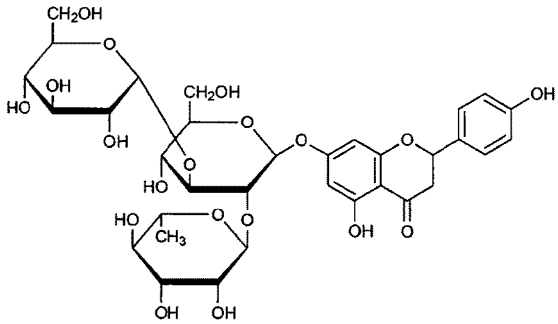

Within the scope of "glycosyl naringenin" as referred to in the present description, "4'-α-monoglucosyl naringin" (Chemical Formula 4 shown below), which has a structure composed of a naringin and a glucose conjugated to the C(4') hydroxyl group of the naringin by α-glycosidic linkage; "3", 4'-α-diglucosyl naringin" (Chemical Formula 5 shown below), which has a structure composed of a naringin and two glucoses, one conjugated to the C(3") hydroxyl group and another conjugated to the C(4') hydroxyl group of the naringin by α-glycosidic linkage; "prunin" (Chemical Formula 6 shown below), which has a structure composed of a naringenin and a β-glucose conjugated to the C(7) hydroxyl group of the naringenin; and further "narirutin" (Chemical Formula 7 shown below), which has a structure composed of a naringenin and a rutinose (α-rhamnosyl (1→6) glucose) conjugated to the C(7) hydroxyl group of the naringenin by β-glycosidic linkage, are also included.

Chemical Formula 4:

[Chem. 4]

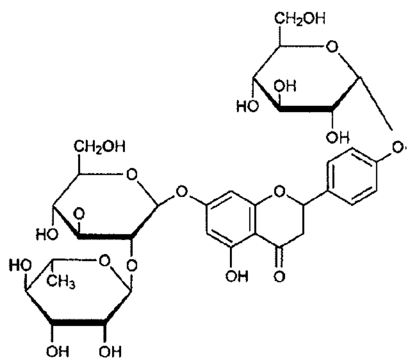

Chemical Formula 5:

[Chem. 5]

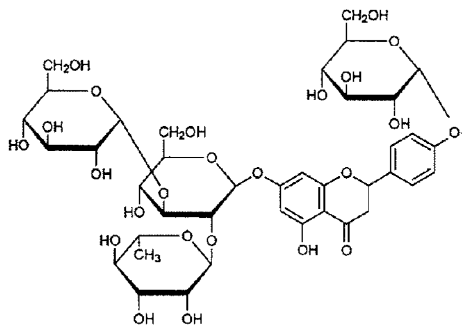

Chemical Formula 6:

[Chem. 6]

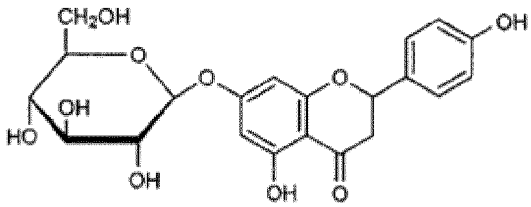

Chemical Formula 7:

[Chem. 7]

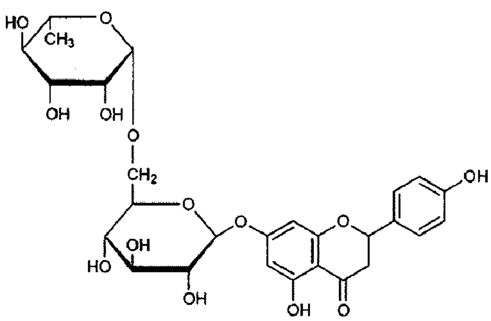

Furthermore, within the scope of "glycosyl naringenin" as referred to in the present description, further glycosylated compounds of the above described prunin, naringin, 3"-α-monoglucosyl naringin, narirutin, 4'-α-monoglucosyl naringin, and 3", 4'-α-diglucosyl naringin are also included. Examples of such glycosylated compounds include but not limited to α-maltosyl naringin, α-maltotriosyl naringin, α-maltotetraosyl naringin, α-maltopentaosyl naringin, α-glucosyl prunin, α-maltosyl prunin, α-maltotriosyl prunin, α-maltotetraosyl prunin, α-maltopentaosyl prunin, α-glucosyl narirutin, α-maltosyl narirutin, α-maltotriosyl narirutin, α-maltotetraosyl narirutin, and α-maltopentaosyl narirutin.

These glycosyl naringenins may be prepared by using an enzyme or by chemical synthesis. Preferably, glycosyl naringenins may be prepared by a method using an enzyme. If necessary, glycosyl naringenins may be prepared by a fermentation method, or a method using an enzyme and chemical synthesis in combination. For producing glycosyl naringenins, an enzymatic method using a glycosyltransferase may be advantageous from the viewpoint of economic efficiency. For example, as a glycosyl naringenin, a series of 3"-α-glycosyl naringins having a glycosyl group with a glucose polymerization degree usually in the range of 1 to 5 can be obtained at high yield, by allowing a glycosyltransferase, such as α-glucosidase, cyclomaltodextrin glucanotransferase, and α-amylase to act on a naringin in the presence of an α-glucosyl saccharide compound, such as a partial starch hydrolyzate and maltooligosaccharide, as disclosed in Japanese Patent Application Publication No. H04-13691, Japanese Patent Application Publication No. 2007-284393, etc. Furthermore, 3"-α-monoglucosyl naringin may be advantageously prepared by allowing a glucoamylase to act on the series of 3"-α-glycosyl naringins.

If a glycosyl naringenin has a glucosyl moiety consisting of D-glucose as a constituent saccharide, for example, at its transglycosylated portion, the glucose polymerization degree may be appropriately reduced by allowing a glucoamylase to act on the glycosyl naringenin. On the other hand, the glucose polymerization degree of the glycosyl group of a glycosyl naringenin can be advantageously increased by allowing a glycosyltransferase, such as cyclomaltodextrin glucanotransferase, to act on the glycosyl naringenin in the presence of a glycosyl donor, such as a partial starch hydrolyzate. If necessary, a monosaccharide other than D-glucose, disaccharide, oligosaccharide, polysaccharide, and so on, may be also conjugated to a glycosyl naringenin by transglycosylation.

Meanwhile, prunin, which is represented by Chemical Formula 6, may be prepared by removing rhamnose residue from a naringin by allowing a rhamnosidase to act on the naringin, as disclosed, for example, in Japanese Patent Application Publication No. 2007-284393. On the other hand, narirutin may be prepared, for example, by transferring a rhamnose to a prunin by α-1,6 transglycosylation. These glycosyl naringins may be also prepared by using a fermentation method, chemical decomposition method, and chemical synthesis method, if necessary.

In the present invention, the glycosyl naringenin may be used regardless of its origin, production method, purity, and the like. It may not necessarily be highly purified. As long as there is no influence on the intended effect and safety, the glycosyl naringenin may be in the form of a mixture with other substances inherent to its production method, in a partially purified form, or even in an unpurified form. For example, if the glycosyl naringenin is produced by an enzymatic method, a crude enzymatic reaction solution comprising the glycosyl naringenin may be directly used.

As described above, the glycosyl naringenin comprised in the composition according to the present invention may be in the form of a composition, in other words, a glycosyl naringenin mixture. Depending on its production process, the glycosyl naringenin mixture usually comprises, as a main ingredient, one or more selected from (1) naringin and α-glycosyl naringin (e.g., α-glucosyl naringin, etc.), which are the compounds having a naringenin skeleton. The glycosyl naringenin mixture may further comprise (2) a flavonoid, such as diosmin and neoponcirin, and (3) a trace component, such as salts. The glycosyl naringenin mixture may comprise a naringenin, an aglycone of the glycosyl naringenin, to the extent that the intended effect of the present invention is not impaired.

A glycosyl naringenin that may be used in the composition of the present invention may comprise only one kind of a compound that falls within the scope of the above described glycosyl naringenin, or may comprise two or more kinds of compounds that fall within the scope of the glycosyl naringenin. From the viewpoint of a high water solubility, the composition of the present invention may comprise preferably an α-glycosyl naringin as the glycosyl naringenin; more preferably an α-glucosyl naringin as the α-glycosyl naringin; and further preferably one or more selected from 3"-α-monoglucosyl naringin, 3", 4'-α-diglucosyl naringin, and 4'-α-monoglucosyl naringin as the α-glucosyl naringin.

The upper limit of the α-glycosyl naringin content on a dry solid basis in the glycosyl naringenin to be contained in the composition of the present invention is not particularly limited. However, it may be usually 99% by mass or less on a dry solid basis, which can be produced on an industrial scale in a relatively large amount, at relatively low cost, and relatively easily. From the viewpoint of providing the glycosyl naringenin at a lower cost, the upper limit of the α-glycosyl naringenin content on a dry solid basis may be 80% by mass or less or even as low as 60% by mass or less. However, when the α-glycosyl naringin content in the glycosyl naringenin on a dry solid basis is low, it becomes inevitable to use a larger amount of the glycosyl naringenin compared to when using the glycosyl naringenin with a high α-glycosyl naringin content, resulting in the poorer handleability. From this viewpoint, the lower limit of the α-glycosyl naringin content in the glycosyl naringenin may be usually 10% by mass or more, preferably 20% by mass or more, more preferably 30% by mass or more, more preferably 40% by mass or more, and further preferably 50% by mass or more. The same applies to the α-glucosyl naringin content in the glycosyl naringenin.

In a preferred embodiment, a glycosyl naringenin to be comprised in the composition of the present invention may comprise an α-glycosyl naringin, and more preferably 3"-α-monoglucosyl naringin as the α-glycosyl naringin. In this case, the preferred 3"-α-monoglucosyl naringin content in the glycosyl naringenin may be usually 10% by mass or more, preferably 20% by mass or more, more preferably 30% by mass or more, further preferably 40% by mass or more, and further more preferably 50% by mass or more, on a dry solid basis. The upper limit of the 3"-α-monoglucosyl naringin content in the glycosyl naringenin to be comprised in the composition of the present invention may basically less than

100%. From the viewpoint of providing the composition of the present invention at a lower cost, the 3"-α-monoglucosyl naringin content may be usually 99% by mass or less, which can be produced on an industrial scale in a relatively large amount, at relatively low cost, and relatively easily, and more preferably as low as 70% by mass or lower.

Next, component (B) will be explained.

Pinene, which may be used in the composition of the present invention, is a type of monoterpene represented by the molecular formula $C_{10}H_{16}$, and is found, for example, in turpentine oil. Pinene include α-pinene and β-pinene. Each of α-pinene and β-pinene has its d-form and l-form. Pinene that may be used in the composition of the present invention may be any one of these pinenes or a mixture thereof.

Sabinene is a type of monoterpene represented by the molecular formula $C_{10}H_{16}$, and is found, for example, in juniper oil. Sabinene has its d-form and l-form. Sabinene that may be used in the composition of the present invention may be either one of these or a mixture thereof.

Camphene is a type of monoterpene represented by the molecular formula $C_{10}H_{16}$, and is found, for example, in rosemary oil and valerian oil. Camphene has its d-form and l-form. Camphene that may be used in the composition of the present invention may be either one of these or a mixture thereof.

Valencene is a type of sesquiterpene represented by the molecular formula $C_{15}H_{24}$, and is found, for example, in citrus plants, such as grapefruit and Valencia orange.

β-Caryophyllene is a type of sesquiterpene represented by the molecular formula $C_{15}H_{24}$, and is found, for example, in essential oils derived from plants of *Myrtaceae* family, clove oil, and cinnamon oil.

β-Ionone is represented by the chemical formula $C_{13}H_{20}O$ and is also called β-ionone. β-Ionone is found, for example, in essential oils of plants of *Rutaceae* family. β-Ionone can be also chemically synthesized by condensing citral and acetone and treating the resultant with acid.

Methyl anthranilate is a nitrogen-containing compound represented by the molecular formula $C_8H_9NO_2$ and is also called methyl 2-aminobenzoate. Methyl anthranilate is known to be contained in, for example, jasmine and grapes. Methyl anthranilate may be also chemically synthesized by dehydration condensation of anthranilic acid and methanol.

Methyl N-methyl anthranilate is a nitrogen-containing compound represented by the molecular formula $C_9H_{11}NO_2$ and is also called methyl N-methyl-2-aminobenzoate. Methyl N-methyl anthranilate is known to be present, for example, in mandarin orange oil. Methyl N-methyl anthranilate may be also chemically synthesized by dehydration condensation of N-methyl anthranilic acid and methanol.

The above-described pinene, camphene, sabinene, valencene, β-caryophyllene, β-ionone, methyl anthranilate, and methyl N-methyl anthranilate, which may be used in the present invention, may be the one produced by chemical synthesis or the like, or the one obtained by extraction from natural products containing these substances. Although it goes without saying that the above-described components that may be used in the composition of the present invention may be the one isolated from natural products or the one that is highly purified, they may not necessarily be isolated and/or purified components. For example, an essential oil obtained from a natural product containing pinene, camphene, sabinene, valencene, β-caryophyllene, β-ionone, methyl anthranilate, or methyl N-methyl anthranilate may be used instead of the isolated or purified components.

On the other hand, thymol, which may be used in the composition of the present invention, is a compound represented by Chemical Formula 8 shown below, and is a type of monoterpene represented by the molecular formula $C_{10}H_{14}O$. Thymol is known to have the bactericidal effect and the blood flow improving effect.

Chemical Formula 8:

[Chem. 8]

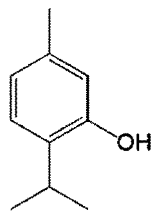

Thymol may be obtained, for example, by chemical synthesis or extraction from a natural product containing thymol. Examples of a natural product containing thymol include but not limited to Japanese citrus fruits such as yuzu, and herbs such as thyme, oregano, and savory. For example, yuzu, thyme, oregano, and horse mint are known to be abundant in thymol. Although it goes without saying that thymol may be isolated from these natural products, thymol

that may be used in the composition of the present invention may not necessarily be an isolated thymol. For example, an essential oil isolated from these natural products may be used instead of an isolated thymol.

Meanwhile, nootkatone, which may be used in the composition of the present invention, is a compound represented by Chemical Formula 9 shown below, and is a type of sesquiterpene ketones represented by the molecular formula $C_{15}H_{22}O$. Nootkatone may be in the form of either its d-form or l-form, or also in the form of a mixture such as a racemate. Nootkatone is known to have AMPK (AMP-activated protein kinase) activity.

Chemical Formula 9:

[Chem. 9]

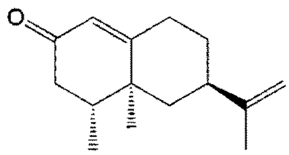

Nootkatone may be obtained, for example, by chemical synthesis, a synthesis using a microorganism, or an extraction from a natural product containing nootkatone. Examples of a natural product containing nootkatone include but not limited to a grapefruit. Nootkatone that may be used in the composition of the present invention may not necessarily be an isolated nootkatone. For example, an essential oil isolated from grapefruit, which is known to contain a relatively large amount of nootkatone, may be used instead of an isolated nootkatone.

Extraction of nootkatone and thymol from natural products may be carried out by appropriately combining an extraction operation using water, hot water, hydrous alcohol, organic solvents, etc., a purification operation using high performance liquid chromatography, column chromatography, etc., and distillation operation. Thymol and/or nootkatone obtained by the above-described synthesis and/or extraction may be preferably of high purity in which contaminants have been removed by a single or multiple steps of purification process, etc. However, a crudely purified product may be also used as long as the intended effect of the present invention is shown.

The above-described component (B) that may be used in the composition of the present invention may be an isomer or a derivative thereof. Examples of a derivative of nootkatone include but not limited to nootkatol, dihydronootkatone, dehydronootkatone, and tetrahydro nootkatone.

On the other hand, examples of an isomer of thymol include but not limited to carvacrol. Examples of a derivative of thymol include but not limited to thymyl acetate, thymol methyl ether, and methyl thymol.

The composition of the present invention comprises component (A) and component (B) as constituents, wherein component (A) is a glycosyl naringenin and component (B) is one or more selected from a group consisting of α-pinene, β-pinene, sabinene, camphene, valencene, β-caryophyllene, β-ionone, methyl anthranilate, methyl N-methyl anthranilate, thymol, and nootkatone. As shown in the later described experiments, the combination of glycosyl naringenin (component (A)) and at least one selected from a group consisting of α-pinene, β-pinene, sabinene, camphene, valencene, β-caryophyllene, β-ionone, methyl anthranilate, methyl N-methyl anthranilate, thymol, and nootkatone (component (B)) significantly increases the secretion of GLP-1. Notably, this effect is superior to the effect obtained when each component is used independently, and can be said to be a synergistic effect that exceeds the addition of the effects obtained when each component is used independently. In particular, since a particularly remarkable synergistic effect can be shown when component (B) is thymol and/or nootkatone, thymol and/or nootkatone may be used particularly preferably as component (B). Accordingly, a composition comprising a glycosyl naringenin together with thymol and/or nootkatone may be a composition for promoting GLP-1 secretion that is particularly effective.

Herein, the term "promoting GLP-1 secretion" as referred to in the present description means that secretion of GLP-1 from GLP-1 secreting cells is promoted in presence of the composition for promoting GLP-1 secretion compared to when the composition is not present, and is a concept including any of the following: promoting GLP-1 secretion in the living body, promoting GLP-1 secretion from L-cells in the intestinal tract, promoting elevation of blood GLP-1 concentration along with GLP-1 secretion in the living body, maintaining elevated GLP-1 concentration, or inhibiting decrease of elevated GLP-1 concentration, in other words, stabilization of blood GLP-1 concentration.

Since GLP-1 is mainly secreted from L-cells that are present in the intestinal tracts, GLP-1 secretion may be promoted by orally administering or orally ingesting a composition comprising component (A) and component (B), wherein component (A) is glycosyl naringenin and component (B) is one or more selected from a group consisting of α-pinene, β-pinene, sabinene, camphene, valencene, β-caryophyllene, β-ionone, methyl anthranilate, methyl N-methyl anthranilate, thymol, and nootkatone. For example, GLP-1 is known to inhibit secretion of glucagon from pancreatic α-cells,

promote secretion of insulin from pancreatic β-cells, and act on central nervous systems, via GLP-1 receptors that are expressed in many organs and tissues, such as pancreatic α-cells, pancreatic β-cells, central nervous system, and stomach. Accordingly, in a preferred embodiment, the composition for promoting GLP-1 secretion of the present invention may be used for controlling various physiological conditions, for example, for preventing and/or improving lifestyle-related diseases, such as obesity and diabetes, improving insulin resistance, suppressing appetite in the central nervous system, delaying gastric excretion in the gastrointestinal tracts, promoting digestion after meal, improving gastric heaviness and promoting gastric acid secretion, or enhancing energy metabolism.

The composition of the present invention may contain any amount of component (A), namely, the glycosyl naringenin, but the total glycosyl naringenin content in the composition may be usually 0.0001% by mass or more, preferably 0.001% by mass or more, preferably 0.002% by mass or more, more preferably 0.02% by mass or more, and further preferably 0.2% by mass or more.

The composition of the present invention may contain component (A), i.e., a glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, at any ratio, as long as the intended effect of the present invention can be obtained. However, from the viewpoint of the synergistic effect of component (A) and component (B), the ratio in terms of the molar ratio (A/B) of component (A) and component (B) may be in the range of 10,000:1 to 1:10, for example, preferably 4,000:1 to 1:10, more preferably 3,731:1 to 1:10, more preferably 1,000:1 to 1:10, more preferably 1,000:1 to 1:1, further preferably 200:1 to 1:1, further preferably 100:1 to 1:1, further more preferably 50:1 to 1:1, and particularly preferably 10:1 to 1:1. When component (B) is thymol, from the viewpoint of the synergistic effect of component (A) and component (B), the ratio in terms of the molar ratio (A/B) of component (A) and component (B) may be in the range of 10,000:1 to 1:10, preferably 4,000:1 to 1:10, more preferably 3,731:1 to 1:10, more preferably 1,000:1 to 1:10, more preferably 1,000:1 to 1:5, more preferably 500:1 to 1:5, further preferably 200:1 to 1:1 and further preferably 100:1 to 1:1.When component (B) is nootkatone, from the viewpoint of the synergistic effect of component (A) and component (B), the ratio in terms of the molar ratio (A/B) of component (A) and component (B) may be in the range of 1,000:1 to 1:5, preferably 500:1 to 1:5, more preferably 333:1 to 1:5, more preferably 200:1 to 1:5, more preferably 100:1 to 1:5, more preferably 50:1 to 1:5, further preferably 10:1 to 1:5, further preferably 3:1 to 1:5, further preferably 1:1 to 1:5, and particularly preferably 10:1 to 1:1. Herein the above ranges expressed using "to" includes both the upper limit and the lower limit.

The amount of administration, route of administration, administration interval, amount of intake, interval of intake of the composition for promoting GLP-1 secretion of the present invention may be appropriately determined depending on the weight, sex, age, condition and other factors of a subject who is administered with or takes the composition. The amount of administration or intake of the composition may be, for example, 0.1 to 500 mg, preferably 1 to 300 mg, more preferably 2 to 200 mg, further preferably 5 to 150 mg per adult (with a body weight of 60 kg) per day in terms of the amount of glycosyl naringenin.

On the other hand, when component (B) includes one or more selected from a group consisting of α-pinene, β-pinene, sabinene, camphene, valencene, β-caryophyllene, β-ionone, methyl anthranilate, and methyl N-methyl anthranilate, the amount of administration or intake of the composition may be, for example, 0.0001 to 10 mg, preferably 0.005 to 5 mg, more preferably 0.001 to 2 mg, further preferably 0.01 to 1 mg per adult (with a body weight of 60 kg) per day in terms of the amount of α-pinene, β-pinene, sabinene, camphene, valencene, β-caryophyllene, β-ionone, methyl anthranilate, or methyl N-methyl anthranilate.

Meanwhile, when component (B) includes thymol, the amount of administration or intake of the composition may be, for example, 0.001 to 10 mg, preferably 0.05 to 5 mg, more preferably 0.01 to 2 mg, further preferably 0.01 to 1 mg per adult (with a body weight of 60 kg) per day in terms of the amount of thymol.

Meanwhile, when component (B) includes nootkatone, the amount of administration or intake of the composition may be, for example, 1 to 2,000 mg, preferably 2 to 1,000 mg, more preferably 5 to 500 mg per adult (with a body weight of 60 kg) per day in terms of the amount of nootkatone.

In the present invention, it may be preferable to administer or intake such an amount of the composition once a day to several times per day, preferably once a day.

The composition of the present invention comprising component (A), i.e., a glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone may be formulated into a single dosage form as a combination preparation. Meanwhile, each component may be also formulated into a separate dosage form so that they can be used simultaneously or separately at intervals.

Since the composition of the present invention has a good water solubility, it can be formulated into various products. Specifically, it may be advantageously used as a material or preparation used by being blended into a flavoring agent, food or beverage, supplement, pharmaceutical, quasi-drug, feed, etc. The flavoring agent, food or beverage, supplement, pharmaceutical, quasi-drug, feed, etc., comprising the composition of the present invention may be very advantageous when administered to or ingested by animals including humans, since it shows the excellent effect of

promoting GLP-1 secretion. It goes without saying that the composition of the present invention may be directly used as a flavoring agent, food or beverage, supplement, pharmaceutical, quasi-drug, feed, etc.

The food or beverage, or supplement that may comprise the composition of the present invention include food products (foods for specified health uses, foods with functional claims) which claim promotion of postprandial GLP-1 secretion and are permitted to be labelled as such as necessary. Food products that are permitted to be labelled with its function can be distinguished from general food products. Examples of the labelling include but not limited to "Improve insulin resistance and lower blood glucose level" and "Suppress postprandial elevation of blood glucose level".

The food or beverage in which the composition of the present invention may be comprised may be basically of any type of foods or beverages, and are not particularly limited to a specific one. It may be provided in any form, and for example, in the form of liquid (including syrup, milk, and suspension), fluid, gel, semi-solid, solid, powder, etc. It may also be a food or beverage that may be prepared in the form of liquid, fluid, gel, semi-solid, solid, powder, etc., just before using. Examples of such foods or beverages include but not limited to alcoholic beverages such as synthetic liquor, brewed liquor, refined sake (seishu), fruit wine, low-malt beer, beer, liqueur, shochu highball (Chu Hi), and medicinal liquor; drinks such as carbonated drinks, soft drinks, tonic water, dairy drinks, smoothies, vegetable juice, fruit juice, sports drinks, soy milk drinks, iron-containing drinks, probiotic drinks, green tea, black tea, herbal tea, cocoa, coffee, non-alcoholic drinks, and energy drinks; stable foods such as rice, porridge, mochi, and bread; noodles such as udon, soba, ramen, and spaghetti; soups such as miso soup and vegetable soup; dairy products such as yogurt and cheese; meat products such as sausages and ham; fish products such as kamaboko, chikuwa, hanpen, and fish sausage; processed seafood products such as canned seafood and dried fish; cooked foods such as nursing care foods and liquid diets and frozen foods obtained by freezing them; confectioneries such as soft candy, hard candy, gummy, cookie, soft cookie, rice cracker, gyuhi, mochi, mousse, bavarois, biscuit, chocolate, chewing gum, caramel, fruit paste, jam, marmalade, cereal bar, protein bar, and energy bar; frozen desserts such as ice cream, sherbet, and gelato; and various seasonings or processed foods such as miso, soy sauce, powdered soy sauce, mayonnaise, dressing, vinegar, noodle soup base, sauce, tomato sauce, curry roux, soup stock, and compound seasoning.

Meanwhile, the food or beverage in which the composition of the present invention may be comprised may contain additives that are acceptable from the viewpoint of food sanitation, if necessary. Examples of such additives include but not limited to a sweetener, acidulant, bitterant, seasoning, flavoring, polysaccharide thickener, emulsifier, preservative, bactericidal or antibacterial agent, pH adjusting agent, tonicity agent, chelating agent, stabilizer, antioxidant, coloring agent, bulking agent, flow improver, excipient, binder, disintegrant, solvent, softener, oil, filler, foaming agent, antifoaming agent, nutrient, luxury goods, taste component, medicinal substance, and an active ingredients other than component (A), i.e., glycosyl naringenin, component (B), i.e., α-pinene, β-pinene, sabinene, camphene, valencene, β-caryophyllene, β-ionone, methyl anthranilate, methyl N-methyl anthranilate, thymol, and nootkatone, which may be comprised in the composition of the present invention. Because naringin, 3"-α-monoglucosyl naringin, 3", 4'-α-diglucosyl naringin, and 4'-α-monoglucosyl naringin, which fall within the scope of the glycosyl naringenin, have a bitter taste, in one embodiment, the composition of the present invention may be advantageously used as a bitterant.

When the composition of the present invention is comprised in a food or beverage, the food or beverage that contains the composition of the present invention may contain any amount of the glycosyl naringenin, and the glycosyl naringenin content in the food or beverage may be appropriately set depending on its usage. There is not particular restriction on the lower limit of the glycosyl naringenin content, but it may preferably 1 ppm or more, more preferably 5 ppm or more, and further preferably 10 ppm or more. On the other hand, there is no particular restriction on the upper limit of the glycosyl naringenin content, neither, but it may be preferably 2,000 ppm or less, more preferably 500 ppm or less, and further preferably 200 ppm or less.

Similarly, the food or beverage that contains the composition of the present invention may contain any amount of sabinene, valencene, β-ionone, methyl N-methyl anthranilate, or thymol, and the content of sabinene, valencene, β-ionone, methyl N-methyl anthranilate, or thymol in the food or beverage may be appropriately set depending on its usage. There is no particular restriction on the lower limit of the content, but it may be preferably 0.001 ppm or more, more preferably 0.01 ppm or more, and further preferably 0.1 ppm or more. There is no particular restriction on the upper limit of the content, but it may be preferably 100 ppm or less, more preferably 10 ppm or less, and further preferably 5 ppm or less.

On the other hand, the food or beverage that contains the composition of the present invention may contain any amount of α-pinene, β-pinene, camphene, β-caryophyllene, methyl anthranilate, or nootkatone, and the content of α-pinene, β-pinene, camphene, β-caryophyllene, methyl anthranilate, or nootkatone in the food or beverage may be appropriately set depending on its usage. There is no particular restriction on the lower limit of the content, but it may be preferably 0.001 ppm or more, more preferably 0.01 ppm or more, further preferably 0.1 ppm or more, and further more preferably 0.5 ppm or more. There is no particular restriction on the upper limit of the content, but it may be preferably 50,000 ppm or less, more preferably 500 ppm or less, further preferably 50 ppm or less, and further more preferably 30 ppm or less.

On the other hand, the supplement in which the composition of the present invention may be comprised refers

to not only dietary supplements and foods with nutrient function claims, etc., for supplementing nutrients, etc., but also health supplements, foods for specified health uses, and foods with function claims that have functions that are useful for maintaining, recovering, and promoting health, etc. These supplements may be provided in any form. For example, they may be provided in the form of liquids (including syrup, milk, and suspension), gels, pastes, tablets, round tablets, capsules (including hard capsules, soft capsules and microcapsules), powders, granules, fine granules and troches. The supplements in these forms may be produced in accordance with a conventional method using appropriate excipients, dispersants, disintegrants, binders, lubricants, capsule bases, coating agents, etc. The supplement in which the composition of the present invention may be comprised may further contain additives that are acceptable from the viewpoint of food sanitation. With regard to the additives that are acceptable from the viewpoint of food sanitation, they are as already described in the description on foods and beverages.

When the composition of the present invention is comprised in a supplement, the supplement that contain the composition of the present invention may contain any amount of the glycosyl naringenin, and the glycosyl naringenin content in the supplement may be appropriately set depending on its usage. There is no particular restriction on the lower limit of the glycosyl naringenin content, but it may be preferably 1 ppm or more, more preferably 10 ppm or more, further preferably 50 ppm or more, and further more preferably 100 ppm or more. On the other hand, there is no particular restriction on the upper limit of the glycosyl naringenin content, neither, but it may be preferably 50% by mass or less, more preferably 30% by mass or less, further preferably 10% by mass or less, and further more preferably 5% by mass or less.

Meanwhile, the supplement that contains the composition of the present invention may contain any amount of sabinene, valencene, β-ionone, methyl N-methyl anthranilate, or thymol, and the content of sabinene, valencene, β-ionone, methyl N-methyl anthranilate, or thymol in the supplement may be appropriately set depending on its usage. There is no particular restriction on the lower limit of the content, but it may be preferably 0.001 ppm or more, more preferably 0.01 ppm or more, further preferably 0.05 ppm or more, and further more preferably 1 ppm or more. There is no particular restriction on the upper limit of the content, but it may be preferably 10% by mass or less, more preferably 5% by mass or less, further preferably 1% by mass or less, and further more preferably 0.1% by mass or less.

On the other hand, the supplement that contains the composition of the present invention may contain any amount of α-pinene, β-pinene, camphene, β-caryophyllene, methyl anthranilate, or nootkatone, and the content of α-pinene, β-pinene, camphene, β-caryophyllene, methyl anthranilate, or nootkatone may be appropriately set depending on its usage. There is no particular restriction on the lower limit of the content, but it may be preferably 0.001 ppm or more, more preferably 0.01 ppm or more, further preferably 0.05 ppm or more, and further more preferably 1 ppm or more. There is no particular restriction on the upper limit of the content, but it may be preferably 10% by mass or less, more preferably 5% by mass or less, further preferably 1% by mass or less, and further more preferably 0.1% by mass or less.

Next, the flavoring agent in which the composition of the present invention may be comprised is a substance that is used to impart flavor or fragrance to a food or beverage and cosmetic product, etc. Such a flavoring agent may be any type of flavoring agent. For example, flavoring agents are classified, depending on their origin, into natural flavoring agents, synthetic flavoring agents, and blended flavoring agents that are obtained by blending one or more of natural flavoring agents and/or synthetic flavoring agents. The composition of the present invention may be comprised in any of natural flavoring agents, synthetic flavoring agents, and blended flavoring agents. Herein, examples of natural flavoring agents include but not limited to plant-based flavoring agents obtained from plant-derived raw materials, animal-based flavoring agents obtained from animal-derived raw materials, such as musk, civet, castoreum, and ambergris. Natural flavoring agents such as plant-based flavoring agents and animal-based flavoring agents may be obtained by production methods, such as squeezing, distillation, and extraction. On the other hand, synthetic flavoring agents include but not limited to isolated flavoring agents, which are isolated from animal and plant oils by distillation, extraction, etc., semi-synthetic flavoring agents, which are synthesized using isolated flavoring agents as raw materials, and synthetic flavoring agents in the narrow sense, which are chemically synthesized mainly using petrochemical-derived chemical products as raw materials.

Among flavoring agents, a flavoring agent used for a food or beverage is called "food flavoring" or "flavor" (Hereinafter, a flavoring agent used for a food or beverage is simply referred to as "food flavoring"). Meanwhile, a flavoring agent used for a cosmetic product is called "fragrance". Since the composition of the present invention shows the effect of promoting GLP-1 secretion, it may be advantageously used mainly as a food flavoring, which is a flavoring agent used for a food or beverage that is orally ingested.

The flavoring agent in which the composition of the present invention may be comprised may be provided in any form that is appropriate for the intended use. For example, when a flavoring agent in which the composition of the present invention is comprised is a food flavoring, the flavoring agent may be in a form appropriate for a variety of food or beverages in in various forms from liquid to solid. Examples of the form of the flavoring agent include but not limited to a water-soluble flavoring, oil-soluble flavoring, emulsified flavoring, powdered flavoring, and the like.

Meanwhile, when the composition of the present invention is added to a flavoring agent, it may be added by

any method at any stage during preparation of the flavoring agent.

The term "water-soluble flavoring" refers to a flavoring agent that is water soluble and in a liquid form. Examples of the water-soluble flavoring include but not limited to essence, recovered aroma, and extract. Water-soluble flavorings may be used for foods or beverages, such as foods with a high water content, beverages, jellies, chilled or frozen confectioneries. Although the blending amount of water-soluble flavorings to be used in foods or beverages varies depending on the type, dosage form, and purpose of use of the foods or beverages, it may be usually in the range of 0.001 to 5%, and preferably in the range of 0.01 to 1%, for example.

Essences are water-soluble flavorings in which a flavor component is dissolved in a water-soluble solvent, such as alcohol, propylene glycol, or glycerin. For example, essences are prepared by extracting and dissolving materials (flavor bases) containing a flavor component, such as essential oils obtained by stream distillation or squeezing from natural products such as flowers, buds, leaves, and stems, using water-containing ethyl alcohol (ethanol), etc. As described above, when the composition of the present invention is added to a flavoring agent, it may be added by any method at any stage during preparation of the flavoring agent. But, when the composition of the present invention is blended for purpose of obtaining essences, component (A), i.e., glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, may be added to and dissolved in a solvent used for extraction, such as water-containing alcohols, in advance, or may be added to and dissolved in an extract obtained as a result of extraction. Thereby, essences containing component (A) and component (B) can be obtained. Herein, component (B) may be added together with component (A) (glycosyl naringenin) or may be added separately. Or, a flavoring agent, fruit juice, vegetable juice, plant-based materials, etc., containing component (B), may be used as a raw material so that component (B) is extracted to the resulting flavoring agent. When more than two kinds of components that fall within the scope of component (B) are used, the more than two components may be added simultaneously or separately.

The water-containing ethanol used for preparing essences may contain any amount of ethanol, but the amount of ethanol may be preferably in the range of 20 to 99 w/w%, more preferably 30 to 80 w/w%, and further preferably 40 to 70 w/w%. The water-containing ethanol may contain a component other than water and ethanol. Examples of such a component include but not limited to glycerin, propylene glycol, etc.

Water-soluble recovered aromas are water-soluble flavorings that can be obtained by concentrating fruit juices, vegetable juices, plant-based materials, meat and seafood extracts, etc., by evaporation method, membrane concentration method, etc. When the composition of the present invention is added to recovered aromas, component (A), i.e., glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, may be directly added to and dissolved in recovered aromas to obtain recovered aromas with even more excellent aroma. Herein, component (B) may be added together with component (A) (glycosyl naringenin) or may be added separately. When more than two kinds of components that fall within the scope of component (B) are used, the more than two components may be added simultaneously or separately.

Extracts are water-soluble flavorings that can be obtained by extracting animal or plant-based materials with water or a water-containing solvents, such as water-containing methanol, water-containing ethanol, water-containing acetone, water-containing glycerin, water-containing ethylene glycol, water-containing propylene glycol, etc., having a water content of about 10 to 90%. When the composition of the present invention is added to extracts, component (A), i.e., glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, may be added to a water-soluble water-containing solvent used for extractions in advance; may be added to an extract obtained as a result of extraction; or may be added to a concentrate that is obtained by removing solvents from the extract. Component (A) and component (B) may be added simultaneously or separately. When more than two kinds of components that fall within the scope of component (B) are used, the more than two components may be added simultaneously or separately.

On the other hand, the term "oil-soluble flavoring" refers to a flavoring agent which is in a form dissolved in an oil-soluble solvent, such as a plant oil. Oil-soluble flavorings can be used for foods or beverages, such as foods that are compatible with oils, breads, chocolates, margarines, and cooked foods. Although it may vary depending on the type, dosage form, and purpose of use of foods or beverages, the blending amount of oil-soluble flavorings in foods or beverages may be usually in the range of 0.01 to 10%, and preferably in the range of 0.05 to 5%, for example.

Oil-soluble flavorings may be prepared, for example, by directly extracting a plant-based material containing a flavor component with an oil and/or fat, or by dissolving a flavoring agent in an oil, fat, propylene glycol, glycerin, or the like. When the composition of the present invention is added to oil-soluble flavorings, component (A), i.e., glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, may be added at an appropriate step. By adding a glycosyl naringenin to an oil-soluble flavoring, the oil-soluble

flavoring with good flavor stability and good thermal stability may be obtained. Component (B) may be added together with component (A) or may be added separately. Or, a flavoring agent, fruit juice, vegetable juice, plant-based materials, etc., containing component (B), may be used as a raw material. Component (A) and component (B) may be added simultaneously or separately. When more than two kinds of components that fall within the scope of component (B) are used, the more than two components may be added simultaneously or separately.

The term "emulsified flavoring" refers to a flavoring in which a flavor component is dispersed in water using an emulsifier. Emulsified flavorings may be used for foods and beverages, including beverages, seasonings such as soups, sauces, and dressings; chilled or frozen confectioneries; desserts; retort foods; processed meat foods; and processed seafood foods. Although it may vary depending on the type, dosage form, and purpose of use of foods or beverages, the formulation amount of emulsified flavorings in foods or beverages may be usually in the range of 0.001 to 5%, and preferably in the range of 0.01 to 1 %, for example.

Emulsified flavorings may be prepared, for example, by dispersing an oil phase containing an oil-soluble flavor component into an aqueous phase containing an emulsifier by applying shear force using an emulsification equipment. When the composition of the present invention is added for purpose of obtaining emulsified flavorings, component (A), i.e., glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, may be added to a water phase before emulsification. Or, component (A) and component (B) may be added to the resulting emulsified composition. By adding component (A) in a water phase before emulsification, emulsified flavorings with an excellent emulsion stability may be obtained. Component (B) may be added together with component (A) or may be added separately. Or, a flavoring agent, fruit juice, vegetable juice, plant-based materials, etc., containing component (B), may be used as a raw material. When more than two kinds of components that fall within the scope of component (B) are used, the more than two components may be added simultaneously or separately.

Emulsified flavorings may further contain other components than a flavor component in its oil phase and/or water phase. There are no restrictions on a component that may be comprised in the oil phase but it may be preferably an oil-soluble component. Examples of such an oil-soluble component include but not limited to oil-soluble natural pigments such as β-carotene, paprika pigment, annatto pigment, chlorophyll, and marigold pigment; fat-soluble vitamins such as cod liver oil, vitamin A, vitamin A oil, vitamin D3, vitamin B2 butyrate, and natural vitamin E mixture; animal and plant-derived oils and fats such as soybean oil, rapeseed oil, corn oil, olive oil, coconut oil, safflower oil, sunflower oil, rice oil, beef tallow, lard, and fish oil; plant-derived resins such as rosin, copal, dammar, gum elemi, and ester gum; medium-chain saturated fatty acid triglyceride with 6 to 12 carbon atoms; and specific gravity adjusting agents such as SAIB (sucrose diacetate hexaisobutyrate). Meanwhile, there are no restrictions on a component that may be comprised in the water phase, neither, but it may be preferably a water-soluble component. Examples of such a water-soluble component include but not limited to emulsifiers such as glycerin fatty acid esters, polyglycerin fatty acid esters, sucrose fatty acid esters, sorbitan fatty acid esters, Quillaja saponin, and lecithin; water-soluble polysaccharides such as gum arabic and soybean polysaccharides; polyhydric alcohols such as glycerin, propylene glycol, sorbitol and sugar alcohols; and antioxidants such as ascorbic acid. Usually, the oil phase and the water phase are separately prepared in advance and then mixed and emulsified.

On the other hand, the term "powdered flavoring" refers to a flavoring agent in a powder form. Powdered flavorings may be used, for example, for foods or beverages, such as beverages, powdered beverages, desserts, chewing gums, candies, chocolates, tablets confectioneries, noodles, snacks, processed seafood foods, and retort foods. Although it may vary depending on the type, dosage form, and purpose of use of foods or beverages, the formulation amount of powdered flavorings in foods or beverages may be preferably in the range of 0.01 to 5%, and preferably in the range of 0.1 to 3%, for example.

Powdered flavorings may be typically produced by emulsifying an oil-soluble flavoring agent using an appropriate emulsifier or surfactant and then adding excipients to make them homogenized, or by powderizing a solution obtained by dispersing a water-soluble flavoring agent in an aqueous solution containing an appropriate coating agent or excipient, using a powderizing method, such as spray drying, vacuum drying, freeze drying, and the like. Powdered flavorings as referred to in the present description also include a powdered product that is prepared by adsorbing a flavoring agent to lactose, trehalose, maltose, porous dextrin, modified starch, isomaltodextrin, etc., and powderizing the same; or a powdered product that is prepared by simply mixing a flavoring agent in a powder form with a powdering base such as lactose, trehalose, maltose, and starch; which are known in the art.

Powdered flavorings may be the one that has been secondary processed by granulation methods, such as tumbling granulation, fluidized bed granulation, mixing and stirring granulation, compression granulation, extrusion granulation, hot-melt granulation; and by wax coating methods, as necessary.

In a preferred embodiment, when the composition of the present invention is added for purpose of obtaining powdered flavorings, component (A), i.e., glycosyl naringenin, and component (B), i.e., one or more selected from a group consisting of α-pinene, β-pinene, sabinene, camphene, valencene, β-caryophyllene, β-ionone, methyl anthranilate, methyl N-methyl anthranilate, thymol, and nootkatone, may be added to an excipient in advance, or component (A) may

be added to the resulting powdered flavorings. By adding component (A) in an excipient in advance, powdered flavorings with excellent stability, which is less likely to be oxidized over time, may be obtained. Component (B) may be added together with component (A) or may be added separately. Or, a flavoring agent, fruit juice, vegetable juice, plant-based materials, etc., containing component (B), may be used as a raw material. When more than two kinds of components that falls within the scope of component (B) are used, the more than two components may be added simultaneously or separately.

There is no particular restriction on the lower limit of the glycosyl naringenin content in the thus obtained flavoring agents, but it may be preferably 1 ppm or more, more preferably 10 ppm or more, further preferably 100 ppm or more, and further more preferably 1,000 ppm or more. On the other hand, there is no particular restriction on the upper limit of the glycosyl naringenin content in the flavoring agent, neither, but it may be preferably 99.9% by mass or less, more preferably 50% by mass or less, further preferably 10% by mass or less, and further more preferably 5% by mass or less.

Meanwhile, there is no particular restriction on the lower limit of the content of sabinene, valencene, β-ionone, methyl N-methyl anthranilate, or thymol in the flavoring agents, but it may be preferably 0.01 ppm or more, more preferably 0.1 ppm or more, further preferably 1 ppm or more, and further more preferably 3 ppm or more. There is no particular restriction on the upper limit of the content of sabinene, valencene, β-ionone, methyl N-methyl anthranilate, or thymol in the flavoring agents, neither, but it may be preferably 10% by mass or less, more preferably 5% by mass or less, further preferably 1% by mass or less, and further more preferably 0.3% by mass or less.

On the other hand, there is no particular restriction on the lower limit of the content of α-pinene, β-pinene, camphene, β-caryophyllene, methyl anthranilate, or nootkatone in the flavoring agents, but it may be preferably 0.01 ppm or more, more preferably 0.1 ppm or more, further preferably 1 ppm or more, and further more preferably 2 ppm or more. There is no particular restriction on the upper limit of the content of α-pinene, β-pinene, camphene, β-caryophyllene, methyl anthranilate, or nootkatone in the flavoring agents, neither, but it may be preferably 10% by mass or less, more preferably 5% by mass or less, further preferably 3% by mass or less, and further more preferably 1% by mass or less.

On the other hand, the composition of the present invention may be incorporated into pharmaceuticals or quasi-drugs that are applied to humans and other animals, including rats, mice, rabbits, sheep, pigs, cows, cats, dogs, monkeys, etc. The pharmaceuticals and quasi-drugs may be administered by any methods, including oral administration and parenteral administration. The route of administration may be appropriately selected depending on the species, age, body weight, etc., of the subject to which the composition of the present invention is to be used.

The composition of the present invention may be incorporated into pharmaceuticals and quasi-drugs in any form. For example, the pharmaceuticals and quasi-drugs may be in the form of a liquid (including a syrup, milk, and suspension), fluid, gel, semi-solid, solid, tablet, round tablet, capsule (including a hard capsule, soft capsule, and microcapsule), powder, granule, fine granule, or troche. It may also be incorporated into pharmaceuticals and quasi-drugs that are prepared into the form of a liquid (including a syrup, milk, and suspension), fluid, gel, semi-solid, solid, tablet, round tablet, capsule (including a hard capsule, soft capsule, and microcapsule), powder, granule, fine granule, troche, etc., just before using. Examples of specific forms of the pharmaceuticals and quasi-drugs for oral administration include but not limited to solid formulations such as tablets, coated tablets, granules, powders, round tablets, troche, and capsules (including hard capsule, soft capsule and microcapsule); and liquid formulations such as elixirs, syrups, and suspensions. Meanwhile, examples of specific forms for parenteral administration include but not limited to injections, transdermal preparations, enteral preparations, infusions, external preparations, suppositories, etc.

The pharmaceuticals or quasi-drugs in which the composition of the present invention may be comprised may further contain a pharmaceutically acceptable base or carrier, a pharmaceutically acceptable additive, and a physiologically active ingredient or pharmacologically active ingredient other than the aforementioned active ingredients that may be comprised in the present invention (i.e., glycosyl naringenin, α-pinene, β-pinene, sabinene, camphene, valencene, β-caryophyllene, β-ionone, methyl anthranilate, methyl N-methyl anthranilate, thymol, and nootkatone).

Examples of physiologically active ingredients or pharmacologically active ingredients that may be comprised in the pharmaceuticals or quasi-drugs of the present invention include but not limited to antibiotics such as penicillin, erythromycin, chloramphenicol, tetracycline, streptomycin, and kanamycin sulfate; vitamin preparations such as thiamine, riboflavin, L-ascorbic acid, cod liver oil, carotenoids, ergosterol, and tocopherol; enzyme preparations such as lipase, esterase, urokinase, protease, and β-amylase; extract preparations such as medicinal ginseng extract, softshell turtle (*Trionyx sinensis*) extract, chlorella extract, aloe extract, and propolis extract; solutions containing hormones such as macrophage migration inhibitory factor, colony stimulating factor, insulin, growth hormone, prolactin, erythropoietin, and follicle-stimulating hormone; and biologics.

The composition of the present invention may be also formulated into feeds for livestock, poultry, pet, and the like. Examples of such feeds include but not limited to livestock feeds for cows, pigs, chickens, sheep, horses, etc.; feeds for small animals such as rabbits, rats, mice, etc.; and pet foods for dogs, cats, small birds, etc. The feeds may be provided in any form, including but not limited to liquid, powder, pellet, flake, or mash. The feeds may further contain, if necessary, other feed materials, such as meats, proteins, cereals, brans, lees, sugars, vegetables, vitamins, minerals,

gelling agents, shape retention agents, pH adjusting agents, seasonings, preservatives, nutritional supplements, etc., and active ingredients other than those of the composition of the present invention.

Depending on the intended use, the composition of the present invention may contain other materials, such as sweeteners, acidulants, bitterants, spices and herbs, seasonings, food flavorings, polysaccharide thickeners, emulsifiers, preservatives, bactericidal or antibacterial agents, pH adjusting agents, tonicity agents, chelating agents, stabilizers, antioxidants, coloring agents, vitamins, excipients, and active ingredients other than those of the composition of the present invention. It may be also possible to provide the composition of the present invention as an intermediate product, a premix, etc., in the form of a composition in combination with these components. When the composition of the present invention is provided as a flavoring agent, food or beverage, supplement, quasi-drug, pharmaceutical, feed, or intermediate product or premix thereof, other materials that may be added to the composition depending on its intended use are exemplified below. But the materials that may be added to the composition of the present invention are not limited to these examples.

Examples of sweeteners (including saccharides) include but not limited to monosaccharides, disaccharides, oligosaccharides, sugar alcohols, and high intensity sweeteners. More specifically, monosaccharides such as glucose, fructose, galactose, arabinose, xylose, rhamnose, ribose, isomerized liquid sugar, and N-acetylglucosamine; disaccharides such as sucrose, maltose, trehalose, palatinose (isomaltulose), lactulose, and lactose; oligosaccharides such as α-cyclodextrin, β-cyclodextrin, γ-cyclodextrin, isomalto-oligosaccharides (e.g., isomaltose, isomaltotriose, panose, etc.), α-glycosyl trehalose, α-glycosyl sucrose, oligo-N-acetylglucosamine, lactosucrose, galactosyllactose, galactopyranosyl (β1-3) galactopyranosyl (β1-4) glucopyranose, galactopyranosyl (β1-3)glucopyranose, galactopyranosyl (β1-6) galactopyranosyl (β1-4) glucopyranose, galactopyranosyl (β1-6) glucopyranose, xylooligosaccharides (e.g., xylotriose, xylobiose, etc.), gentiooligosaccharides (e.g., gentiobiose, gentiotriose, gentiotetraose, etc.), stachyose, theande oligo, nigerooligosaccharide (e.g., nigerose, etc.), palatinose oligosaccharide, palatinose syrup, fucose, fructooligosaccharides (e.g., kestose, nystose, etc.), fructofuranosylnystose, polydextrose, maltosyl β-cyclodextrin, maltooligosaccharides (e.g., maltotriose, maltotetraose, maltopentaose, maltohexaose, maltoheptaose, etc.), raffinose, erlose, soybean oligosaccharide, invert sugar, starch syrup, etc; sugar alcohols such as sorbitol, maltitol, isomaltitol, maltotriitol, erythritol, xylitol, glycerol, palatinit, mannitol, lactitol, reduced isomaltooligosaccharides, reduced xylooligosaccharides, reduced gentiooligosaccharides, reduced maltose syrup, and reduced starch syrup; high intensity sweeteners such as dihydrochalcone, α-glucosyltransferase-treated stevia, aspartame, acesulfame potassium, alitame, advantame, neotame, L-aspartyl-L-phenylalanine methyl ester, thaumatin, monellin, saccharin, cyclamate, sucralose, stevia extract, stevia powder, licorice extract (glycyrrhizin), dulcin, Tenryocha extract, Nigeria berry extract, fructosyltransferase-treated stevia, Lakanka extract, enzyme-treated licorice, and enzymatically degraded licorice; and others such as honey, maple sugar, fruit juice, fruit juice concentrate, etc.

Examples of acidulants include but not limited to benzoic acid, adipic acid, sorbic acid, citric acid, trisodium citrate, glucono-delta-lactone, gluconic acid, potassium gluconate, sodium gluconate, succinic acid, monosodium succinate, disodium succinate, sodium acetate, DL-tartaric acid, L-tartaric acid, sodium DL-tartrate, sodium L-tartrate, lactic acid, sodium lactate, acetic acid, fumaric acid, monosodium fumarate, DL-malic acid, sodium DL-malate, phosphoric acid, malic acid, itaconic acid, α-ketoglutaric acid, phytic acid, disodium dihydrogen pyrophosphate, acid sodium metaphosphate, vinegar, fruit juice, etc..

Examples of bitterants include but not limited to iso-α bitter acid, caffeine (extract), quinine, quassin, *Trametes versicolor* extract, Cinchona extract, *Phellodendron amurense* extract, *Gentiana lutea* extract, spice and herb extract, *Jamaica quassia* extract, theobromine, *Picrasma quassioides* extract, *Artemisia absinthium* extract, tea extract, *Isodon japonicus* extract, *Agaricus subrufescens* extract, *Tinospora cordifolia* (Boraphet) extract, methylthioadenosine, *Litchi chinensis* extract, olive tea, *Citrus aurantium* extract, *Humulus lupulus* (hop) extract, *Artemisia indica* extract, etc.

Examples of spices and herbs include but not limited to *Trachyspermum ammi* (ajwain), *Angelica archangelica* (angelica), *Pimpinella anisum* (anise), *Curcuma longa* (turmeric), *Allium cepa* (onion), *Pimenta dioica* (allspice), *Origanum vulgare* (oregano), *Allium sativum* (garlic), cardamom, *Carum carvi* (caraway), *Syzygium aromaticum* (clove), *Capparis spinosa* (caper), *Piper nigrum* (pepper) (red pepper, black pepper, white pepper, green pepper, etc.), *Crocus sativus* (saffron), *Zingiber officinale* (ginger), *Sinapis alba* (white mustard), *Coriandrum sativum (coriander), Salvia officinalis* (sage), *Zanthoxylum piperitum* (Japanese pepper), *Cinnamomum* (cinnamon), *Illicium verum* (star anise), *Armoracia rusticana* (horseradish), *Apium graveolens var. dulce* (celery), *Sesamum indicum* (sesame), *Thymus* (thyme), *Artemisia dracunculus* (Tarragon), *Curcuma longa* (turmeric), chili pepper, *Allium schoenoprasum var. schoenoprasum* (chives), *Citrus reticulata* peel, *Capsicum annuum* (chili pepper), nutmeg, *Ocimum basilicum* (basil), *Capsicum annuum grossum* (paprika), *Petroselinum crispum* (parsley), *Vanilla planifolia* (vanilla), *Mentha x piperita L.* (peppermint), *Origanum majorana* (marjoram), mustard, *Zingiber mioga* (myoga), mace, *Cymbopogon citratus* (lemongrass), *Salvia Rosmarinus* (rosemary), and laurel.

Examples of seasonings include but not limited to L-asparagine, L-aspartic acid, sodium L-aspartate, DL-alanine, L-alanine, L-arginine, L-arginine-L-glutamate, L-isoleucine, glycine, L-glutamine, L-glutamic acid, Potassium L-glutamate, calcium L-glutamate, sodium L-glutamate, magnesium L-glutamate, L-cystine, L-serine, taurine (extract), L-tyrosine, L-theanine, DL-tryptophan, L-tryptophan, DL-threonine, L-threonine, L-valine, L-histidine, L-histidine hydrochloride, L-hydroxyproline, L-phenylalanine, L-proline, betaine, DL-methionine, L-methionine, L-lysine, L-lysine-L-aspartate, L-lysine hydrochloride, L-lysine-L-glutamate, L-leucine, disodium 5'-inosinate, disodium 5'-uridylate, disodium 5'-guanylate, disodium 5'-cytidylate, calcium 5'-ribonucleotide, disodium 5'-ribonucleotide, monopotassium citrate, tripotassium citrate, calcium citrate, trisodium citrate, succinic acid, monosodium succinate, disodium succinate, sodium acetate (crystal), sodium acetate (anhydrous), sodium hydrogen DL-tartrate, sodium hydrogen L-tartrate, sodium DL-tartrate, sodium L-tartrate, calcium lactate, sodium lactate, monosodium fumarate, sodium DL-malate, potassium chloride, sodium chloride-decreased brine (saline lake), crude potassium chloride (sea water), whey salt, tripotassium phosphate, dipotassium hydrogen phosphate, potassium dihydrogen phosphate, disodium hydrogen phosphate (crystal), disodium hydrogen phosphate (anhydrous), sodium dihydrogen phosphate (crystal), sodium dihydrogen phosphate (anhydrous), trisodium phosphate (crystal), trisodium phosphate (anhydrous), and chlorella extract.

Examples of food flavorings include but not limited to citrus flavors, such as orange, lemon, lime, grapefruit, mandarin, tangerine, shiikuwasha, yuzu, mandarin orange, citrus, bergamot, iyokan, kabosu, bitter orange, buntan, kumquat, sudachi, and ponkan flavors; fruit flavors, such as apple, banana, cherry, grape, melon, peach, pineapple, plum, cranberry, and strawberry flavors; beans flavors, such as vanilla, coffee, cocoa, and chocolate flavors; mint flavors, such as peppermint, spearmint, and horsemint flavors; spice flavors, such as allspice, cinnamon, and nutmeg flavors; nut flavors, such as almond, peanut, and walnut flavors; seafood flavors, such as crab, shrimp, and other seafood flavors; and other flavors, such as vegetable, cereal, and seaweed flavors.

Examples of polysaccharide thickeners include but not limited to alginic acid and its salts (e.g. sodium alginate, etc.), propylene glycol alginate, carboxymethylcellulose calcium, sodium carboxymethylcellulose, hydroxypropyl methylcellulose, methylcellulose, sodium polyacrylate, almond gum, gum arabic, xanthan gum, galactomannan (e.g. locust bean gum, guar gum, tara gum, etc.), psyllium seed gum, Artemisia seed gum, sesbania gum, tamarind seed gum, gellan gum, deacylated gellan gum, native gellan gum, carrageenan, glucomannan, konjac powder, macrohomopsis gum, arabinogalactan, elemi resin, karaya gum, dammar resin, tragacanth gum, peach gum, linseed gum, cassia gum, Gleditsia triacanthos gum, agar, gelatin, pectin (e.g. HM pectin, LM pectin, etc.), pullulan, curdlan, tragacanth gum, gum ghatti, arabinogalactan, gum karaya, chitin, welan gum, starches (e.g., starch, sodium carboxymethyl starch, carboxymethyl starch, hydroxypropyl starch, pregelatinized starch, phosphoric acid-crosslinked starch, starch octenyl succinate, starch acetate, etc.), dextrins (e.g. branched dextrin, isomaltodextrin, polydextrose, indigestible dextrin, etc.), dextran, soybean polysaccharides, crystalline cellulose and its preparations, powdered cellulose and its preparations, chitin, chitosan, glucosamine, oligoglucosamine, PGA, porphyran, furcellulan, fucoidan, etc.

Examples of emulsifiers include but not limited to glycerin fatty acid ester, acetic and fatty acid esters of glycerol, lactic and fatty acid esters of glycerol, glycerol succinic acid fatty acid ester, diacetyltartaric and fatty acid esters of glycerol, sorbitan fatty acid ester, sucrose fatty acid ester, sucrose acetate isobutyrate, polyglycerol ester of fatty acid, polyglycerol esters of interesterified ricinoleic acid, propylene glycol fatty acid ester, calcium stearoyl lactylate, sodium stearoyl lactylate, polyoxyethylene sorbitan monoglyceride, lecithin, and lysolecithin.

Examples of preservatives, bactericidal agents or antibacterial agents include but not limited to polidronium chloride, alkyldiaminoethylglycine hydrochloride, sodium benzoate, ethanol, benzalkonium chloride, benzethonium chloride, chlorhexidine gluconate, chlorobutanol, sorbic acid, potassium sorbate, sodium dehydroacetate, methyl parahydroxybenzoate, ethyl parahydroxybenzoate, propyl parahydroxybenzoate, butyl parahydroxybenzoate, oxyquinoline sulfate, phenethyl alcohol, benzyl alcohol, biguanide compounds (e.g., polyhexamethylene biguanide or its hydrochloride salt, etc.), Glokill (manufactured by Rhodia S.A.)

Examples of pH adjusting agents include but not limited to hydrochloric acid, sodium hydroxide, potassium hydroxide, calcium hydroxide, magnesium hydroxide, triethanolamine, monoethanolamine, diisopropanolamine, sulfuric acid, and phosphoric acid.

Examples of tonicity agents include but not limited to sodium bisulfite, sodium sulfite, potassium chloride, calcium chloride, sodium chloride, magnesium chloride, potassium acetate, sodium acetate, sodium hydrogen carbonate, sodium carbonate, sodium thiosulfate, magnesium sulfate, disodium hydrogen phosphate, sodium dihydrogen phosphate, potassium dihydrogen phosphate, glycerin and propylene glycol.

Examples of chelating agents include but not limited to ascorbic acid, tetrasodium edetate, sodium edetate, and citric acid.

Examples of stabilizers include but not limited to trometamol, sodium formaldehyde sulfoxylate (Rongalite), tocopherol, sodium pyrosulfite, monoethanolamine, aluminium monostearate, and glyceryl monostearate.

Examples of antioxidants include but not limited to water-soluble antioxidants such as ascorbic acid, ascorbic acid derivatives (e.g., calcium ascorbate, sodium ascorbate, potassium ascorbate, ascorbic acid phosphate, ascorbic acid 2-phosphate magnesium salt, sodium ascorbic acid phosphate, ascorbic acid 2-glucoside, etc.), sodium bisulfite, sodium sulfite, and sodium thiosulfate.

Examples of coloring agents include but not limited to natural pigments such as gardenia yellow, matcha pigment, cochineal extract, gardenia yellow pigment, gardenia blue pigment, flavonoid pigment, caramel pigment, β-carotene, carotenoid pigment, paprika pigment, chlorophyll, and charcoal; and synthetic coloring agents such as Red No. 2, Red No.3, Red No. 104, Red No. 105, Red No. 106, Yellow No. 4, Yellow No. 5, Blue No. 1 and titanium dioxide.

Examples of vitamins include but not limited to vitamin B1 such as thiamine hydrochloride, thiamine nitrate, and cocarboxylase (thiamine pyrophosphate); vitamin B1 derivatives such as fursultiamine, prosulthiamine, octotiamine, thiamine disulfide, bisbentiamine, bisbutiamine, bisibuthiamine, benfotiamine, dicethiamine, cycotiamine and their salts; vitamin B6 such as pyridoxine, pyridoxamine, pyridoxal, pyridoxine phosphate, pyridoxal phosphate, pyridoxamine phosphate and their salts; and vitamin B12 such as cobamamide, cyanocobalamin, hydroxocobalamin acetate, mecobalamin, and their salts. Furthermore, examples of other vitamin Bs include nicotinic acid and its salts, nicotinamide, pantothenic acid and its salt, panthenol, pantethine, folic acid and its salt, and biotin.

Examples of excipients include but not limited to polysaccharides or their derivatives, such as pullulan, carrageenan, xanthan gum, carboxymethylcellulose, cellulose, hemicellulose, gum arabic, guar gum, pectin, chitin, agarose, dextran, dextrin, isomaltodextrin, amylose, and starch including modified starch; polymers such as proteins including gelatin, casein, etc.; saccharides such as sorbitol, mannitol, maltitol, sucrose, maltose, lactose, α, α-trehalose, α, β-trehalose, gum arabic, cornstarch, and crystalline cellulose; inorganic substances such as aluminum hydroxide, calcium hydroxide, magnesium hydroxide, barium hydroxide, calcium sulfate, calcium sulfite, calcium carbonate, silica, calcium silicate, basic magnesium carbonate, kaolin, and talc.

The present invention is explained in further detail with reference to the following experiments. However, the scope of the present invention is not limited by these experimental examples. Appropriate alterations and modifications are possible within the technical scope of the present invention.

<Experiment 1: Confirmation of GLP-1 Secretion-Promoting Effect of Glycosyl Naringenin and Various Flavor Components>

A 3"-α-monoglucosyl naringin-containing composition is used as a glycosyl naringenin, compositions containing various flavor components were prepared. The thus prepared compositions were applied to human intestinal tract-derived cells, and the amount of GLP-1 secretion was examined.

<Experiment 1-1: Preparation of 3"-α-Monoglucosyl Naringin-containing Composition>

Glycosyl naringenin was prepared in accordance with the method described in Example 1 of Japanese Patent Application Publication No. 2002-199896. Specifically, 50 g of naringin and 200 g of dextrin with Dextrose Equivalent (DE) of 8 were dissolved in 500 mL of water under heating, and pH of the resultant solution was adjusted to 7.0 by addition of 2N sodium hydroxide aqueous solution. After the adjustment of pH, cyclodextrin glucanotransferase (manufactured by Hayashibara Co., Ltd., Okayama, Japan) from *Bacillus stearothermophilus* in an amount of 15 units/g-dextrin was added to the solution, and the solution was subjected to a reaction for 48 hours at 68°C. After the reaction, the solution was heated to inactivate the enzyme and filtered to obtain a reaction solution comprising α-glycosyl naringin. Next, pH of this reaction solution comprising α-glycosyl naringin was adjusted to 4.5. Then, glucoamylase (Product Name: Glucozyme; manufactured by Amano Enzyme Inc., Japan) in an amount of 100 units/g-dextrin was added to the reaction solution, and the reaction solution was subjected to a reaction for 24 hours at 55°C so that α-glucosyl naringin is produced from α-glycosyl naringin. As shown in Japanese Patent Application Publication No. 2002-199896, the thus produced α-glucosyl naringin contained 3"-α-monoglucosyl naringin and unreacted naringin, and in addition 3", 4'-α-diglucosyl naringin and 4'-α-monoglucosyl naringin as other glycosylated naringins. Therefore, in order to increase the amount of 3"-α-monoglucosyl naringin and naringin in the composition, α-glucosidase (Product Name: Transglucosidase L "Amano", manufactured by Amano Enzyme Inc., Japan) in an amount of 1 mL/g-α-glycosyl naringin was added to the reaction solution, and the reaction solution was subjected to a reaction for 24 hours at 55°C so that 3",4'-α-diglucosyl naringin and 4'-α-monoglucosyl naringin were decomposed to produce a composition comprising 3"-α-monoglucosyl naringin and naringin. After the reaction, the resultant reaction solution was heated to inactivate the enzyme and then filtered. The obtained filtrate was passed through a column packed with a porous synthetic adsorbent at a space velocity (SV) of 2 so that 3"-α-monoglucosyl naringin and naringin in the solution were made adsorbed to the column. The column was washed with water to wash out glucose, salts, etc., which did not adsorb to the column. Thereafter, the column was fed with an aqueous ethanol solution and the concentration of ethanol in the aqueous ethanol solution was increased in a stepwise manner, to have an eluent comprising 3"-α-monoglucosyl naringin and naringin. The obtained eluent was concentrated in vacuo and powderized by spray-drying, to obtain a 3"-α-monoglucosyl naringin composition. The HPLC analysis revealed that the 3"-α-monoglucosyl naringin composition was composed of 70% of 3"-α-monoglucosyl naringin and 30% of naringin in terms of the molar ratio (equivalent to the molar ratio of naringenin aglycone). The HPLC analysis was performed under the following condition.

<Condition for HPLC Analysis>

Column: "CAPCELL PAK C18 UG 120" (manufactured by Shiseido Company, Limited, Japan)
Eluent: Water/Acetonitrile/Acetic acid = 80/20/0.01 (v/v/v)
Detection: UV 280 nm
Temperature: 40°C
Flow rate: 0.8 mL/min

<Experiment 1-2: Preparation of High-Purity 3"-α-Monoglucosyl Naringin>

The 3"-α-monoglucosyl naringin composition obtained in Experiment 1-1 was dissolved in a mixed solution of water/acetonitrile/acetic acid at volume ratio of 80/20/0.01 (v/v/v), and passed through a C18 column following the previously described condition for HPLC analysis while using UV/VIS Spectrophotometer (UV 280 nm) as a detector. Thereby, 3"-α-monoglucosyl naringin and naringin were separated, and a fraction containing 3"-α-monoglucosyl naringin was collected. The collected fraction containing 3"-α-monoglucosyl naringin was concentrated in vacuo and powderized by spray-drying to obtain a purified product of high-purity 3"-α-monoglucosyl naringin in a powder form. The HPLC analysis revealed that the purity of 3"-α-monoglucosyl naringin of the obtained purified product was 99.0%.

<Experiment 1-3: Examination of GLP-1 Secretion-Promoting Effect by Combination of mGN and Flavor Component>

GLP-1 secretion-promoting effect was evaluated by the following experiment using NCI-H716 cells (Human intestinal tract-derived cell line; purchased from ATCC).

[Text Substances]

In Experiment 1-3, the following compounds were used as test substances.

<Test Sample>

The purified product of high-purity 3"-α-monoglucosyl naringin (hereinafter referred to as "mGN") obtained by the method described in Experiment 1-2, and, as flavor components, citral, camphene (all manufactured by Sigma-Aldrich, Merck KGaA, Darmstadt, Germany (hereinafter referred to as "Sigma")), citronellal (manufactured by Combi Blocks, Inc., CA, USA), methyl anthranilate, methyl N-methyl anthranilate, nootkatone (manufactured by Tokyo Chemical Industry Co., Ltd., Tokyo, Japan), α-pinene, β-pinene, β-ionone, β-caryophyllene, thymol (manufactured by FUJIFILM Wako Pure Chemical Corporation, Osaka, Japan), sabinene (manufactured by Toronto Research Chemicals, ON, Canada), and valencene (manufactured by Cayman Chemical Company, MI, USA) were used.

NCI-H716 cells were cultured at 37°C with 5% $CO_2$ using RPMI1640 (supplemented with 10% Fetal Bovine Serum, manufactured by Sigma) as a cell culture medium for passage. After culturing, cells were suspended in DMEM medium (supplemented with 10% Fetal Bovine Serum; containing 4.5 mg/mL of glucose; manufactured by Shimadzu Diagnostics Corporation, Tokyo, Japan) and added to a Matrigel (120 μL/well; manufactured by Corning Incorporated, NY, USA)-coated 48-well plate at a cell density of $1.2 \times 10^5$ cells/well. After incubation for 2 days, the cells were washed twice with 20 mM HEPES-containing HBSS (Hank's Balanced Salt Solution) buffer. After removal of the washing buffer, 20 mM HEPES-HBSS (supplemented with 0.01% BSA) containing the above-described test samples was added and the cells were incubated for 2 hours. After incubation, the supernatant was collected into a microcentrifuge tube containing diprotin-A (DPP4 inhibitor, manufactured by Abcam plc., Cambridge, UK) and protease inhibitors (cOmplete™, EDTA-free Protease Inhibitor Cocktail, Roche, Basel, Switzerland; Catalogue No. 1187358001). After centrifugation, the supernatant was stored at -80°C until it was used for the GLP-1 measurement. The amount of GLP-1 in the medium was measured by ELISA method using GLP-1 Active Form Assay Kit (manufactured by Immuno-Biological Laboratories Co, Ltd., Gunma, Japan). On the other hand, it was confirmed that the samples used for the GLP-1 measurement showed no cytotoxicity by measuring lactate dehydrogenase (LDH) activity in the cell culture supernatants. As a positive control for measuring cytotoxicity, the cells added with 0.1% Triton X-100/PBS (-) (Phosphate Buffered Saline) were used. The LDH activity in the cell culture supernatant of the positive control was set as 100% and the ratio to the positive control (LDH activity in the cell culture supernatant of sample/LDH activity in the positive control) was calculated as the cytotoxicity rate. Herein, it was judged that a sample had no cytotoxicity when the cytotoxicity rate was within ±2% compared to that of "control (cells cultured with 20 mM HEPES-HBSS without a test sample, instead of 20 mM HEPES-HBSS containing a test sample; shown as "No Addition" in Table 1)".

The amount of GLP-1 secretion was determined as a relative value to the average value of the GLP-1 secretion amount obtained for a control (cells cultured with 20mM HEPES-HBSS without a test sample instead of 20mM HEPES-HBSS containing a test sample; shown as "No Addition" in Table 1). In Table 1, the GLP-1 secretion amounts when mGN and flavor components were used separately and the GLP-1 secretion amounts when mGN and flavor components were used in combination were shown. As shown in Table 1, the concentration of test samples in the cell culture medium was 2.5 mM for mGN and 0.25 mM for flavor components. The synergistic effect of mGN and flavor components in promoting GLP-1 secretion was judged based on the following criteria.

[Formula]

Synergistic Effect: Theoretical Value < (is less than) Measured Value

$$\text{Theoretical Value} = (A/D-1) \times 100 + (B/D-1) \times 100$$

$$\text{Measured Value} = (C/D-1) \times 100$$

A: GLP-1 secretion amount when mGN was used alone
B: GLP-1 secretion amount when a flavor component was used alone
C: GLP-1 secretion amount when mGN and a flavor component were used in combination
D: GLP-1 secretion amount of Control (No addition)

The theoretical value and the measure value were compared based on the above formula. When the measured value was higher than the theoretical value, it was judged that there was a synergistic effect, which was indicated as "O" in the table.

[Table 1]

| Test No. | Test Sample | | Independent Use | Combination | Synergistic Effect |
|---|---|---|---|---|---|
| Control | No Addition | | 100 | - | - |
| 1-1 | mGN<br>Citral | 2.5 mM<br>0.25 mM | 164<br>246 | 156 | × |
| 1-2 | mGN<br>Citronellal | 2.5 mM<br>0.25 mM | 164<br>113 | 163 | × |
| 1-3 | mGN<br>α-Pinene | 2.5 mM<br>0.25 mM | 164<br>136 | 257 | ○ |
| 1-4 | mGN<br>β-Pinene | 2.5 mM<br>0.25 mM | 164<br>100 | 269 | ○ |
| 1-5 | mGN<br>Sabinene | 2.5 mM<br>0.25 mM | 164<br>101 | 227 | ○ |
| 1-6 | mGN<br>Camphene | 2.5 mM<br>0.25 mM | 164<br>135 | 272 | ○ |
| 1-7 | mGN<br>Valencene | 2.5 mM<br>0.25 mM | 164<br>121 | 234 | ○ |
| 1-8 | mGN<br>β-Caryophyllene | 2.5 mM<br>0.25 mM | 164<br>111 | 229 | ○ |
| 1-9 | mGN<br>β-Ionone | 2.5 mM<br>0.25 mM | 164<br>157 | 264 | ○ |
| 1-10 | mGN<br>Methyl anthranilate | 2.5 mM<br>0.25 mM | 164<br>73 | 274 | ○ |

(continued)

| Test No. | Test Sample | | Independent Use | Combination | Synergistic Effect |
|---|---|---|---|---|---|
| Control | No Addition | | 100 | - | - |
| 1-11 | mGN<br>Methyl N-methyl anthranilate | 2.5 mM<br>0.25 mM | 164<br>73 | 228 | ○ |
| 1-12 | mGN<br>Thymol | 2.5 mM<br>0.25 mM | 164<br>82 | 648 | ○ |
| 1-13 | mGN<br>Nootkatone | 2.5 mM<br>0.25 mM | 164<br>457 | 1,051 | ○ |

As shown in Table 1, the GLP-1 secretion-promoting effect compared to Control was observed when mGN and specific flavor components were used in combination. In detail, while mGN alone promoted GLP-1 secretion approximately 1.6 times, combination of mGN and citral similarly promoted GLP-1 secretion approximately 1.5 times. Since citral alone promoted GLP-1 secretion approximately 2.4 times, it was thought that the GLP-1 secretion-promoting effect shown by citral was rather inhibited by combination with mGN. Meanwhile, when mGN was used in combination with citronellal, no synergistic increase in GLP-1 secretion was observed compared to when mGN or citronellal was used alone. In contrast, when mGN was used in combination with α-pinene, β-pinene, sabinene, camphene, valencene, β-caryophyllene, β-ionone, methyl anthranilate, methyl N-methyl anthranilate, thymol or nootkatone, an increased GLP-1 secretion was observed compared to when mGN or each of the flavor components was used alone, and furthermore, it was judged that there were synergistic effects. Among them, β-pinene, sabinene, methyl anthranilate, methyl N-methyl anthranilate, and thymol showed no GLP-1 secretion promoting effect when they were used alone. However, it was revealed that these flavor components synergistically enhanced the GLP-1 secretion-promoting effect shown by mGN when they were used in combination with mGN. Surprisingly, when mGN and thymol were used in combination, approximately 6.5 times more GLP-1 secretion was observed compared to Control, which was approximately 4 times more even compared to when mGN was used alone. Similarly, when mGN and nootkatone were used in combination, GLP-1 secretion was approximately 10 times more compared to Control and approximately 6 times more compared to when mGN was used alone. The above results revealed that the GLP-1 secretion-promoting effect can be synergistically enhanced when mGN was used in combination with α-pinene, β-pinene, sabinene, camphene, valencene, β-caryophyllene, β-ionone, methyl anthranilate, methyl N-methyl anthranilate, thymol or nootkatone; and that, among them, when mGN was used in combination with β-pinene, sabinene, methyl anthranilate, methyl N-methyl anthranilate, thymol or nootkatone, the more synergistically enhanced GLP-1 secretion promoting effect can be obtained. In particular, it was revealed that when mGN was used in combination with thymol or nootkatone, the remarkably better GLP-1 secretion promoting effect compared to the combination with other flavor components can be obtained.

<Experiment 2: Ratio of mGN and Thymol and its Effect on GLP-1 Secretion-Promoting Effect - Part 1- >

In Experiment 1-3, the particularly good GLP-1 secretion-promoting effect was observed for the combination of mGN and thymol and the combination of mGN and nootkatone. Herein, the GLP-1 secretion-promoting effect shown by the combination of mGN and thymol was studied in more detail. Compositions were prepared by mixing mGN and 0.25 mM of thymol at various molar ratios, and their effects on GLP-1 secretion were examined. The experiment was carried out in the same manner as in Experiment 1-3 except that test samples shown in Table 2 were used. The number of samples (N) for each test sample was 3.

The presence or absence of the synergistic effect by the combination of mGN and thymol was judged based on the formula used in Experiment 1-3. When the measured value was higher than the theoretical value, it was judged that there was a synergistic effect and it was indicated as "O" in the table. Furthermore, a Tukey-Kramer test was performed. When there was a significant difference (Risk Rate $p<0.05$) between the GLP-1 secretion amount of the combination of mGN and thymol (C) and each of the GLP-1 secretion amount of Control (D), mGN alone (A), and thymol alone (B), it was indicated "◎" in the below table.

[Table 2]

| Test No. | Test Sample | | Independent Use | Combination | Molar Ratio (mGN: Thymol) | Synergistic Effect |
|---|---|---|---|---|---|---|
| Control | No Addition | | 100 | - | - | - |
| 2-1 | mGN | 0.025 mM (0.002% by mass) | 45 | 140 | 1:10 | ○ |
| | Thymol | 0.25 mM (0.004% by mass) | 119 | | | |
| 2-2 | mGN | 0.25 mM (0.02% by mass) | 63 | 380 | 1:1 | ◎ |
| | Thymol | 0.25 mM (0.004% by mass) | 82 | | | |
| 2-3 | mGN | 2.5 mM (0.2% by mass) | 121 | 574 | 10:1 | ◎ |
| | Thymol | 0.25 mM (0.004% by mass) | 82 | | | |
| 2-4 | mGN | 25 mM (2% by mass) | 427 | 2,000 | 100:1 | ◎ |
| | Thymol | 0.25 mM (0.004% by mass) | 82 | | | |
| 2-5 | mGN | 50 mM (4% by mass) | 803 | 1,697 | 200:1 | ◎ |
| | Thymol | 0.25 mM (0.004% by mass) | 85 | | | |
| 2-6 | mGN | 150 mM (11% by mass) | 1.912 | 2,124 | 600:1 | ○ |
| | Thymol | 0.25 mM (0.004% by mass) | 85 | | | |

As shown in Table 2, when thymol was used alone at a concentration of 0.25 mM, no GLP-1 secretion-promoting effect was observed and the rather decreased GLP-1 secretion compared to Control was observed for most of the cases. Meanwhile, when 0.025 mM to 150 mM of mGN was added, the GLP-1 secretion was promoted to some extent in a mGN concentration-dependent manner, although mGN was used alone. In contrast, when thymol and mGN were used in combination, GLP-1 secretion was remarkably promoted compared to when they were used separately. In detail, in all of Tests Nos. 2-1 to 2-6, in which the molar ratio of mGN and thymol was in the range of 600:1 to 1:1, the synergistic effect shown by the combination of mGN and thymol was observed. In particular, in Tests Nos. 2-2 to 2-5, in which the formulation ratio of mGN and thymol in a molar ratio was in the range of 200:1 to 1:1, the more effective enhancement in the GLP-1 secretion-promoting effect was observed.

<Experiment 3: Ratio of mGN and Thymol and its Effect on GLP-1 Secretion-Promoting Effect - Part 2 - >

Next, taking Test No. 2-4, which showed the highest GLP-1 secretion-promoting effect in Table 2, as a standard, compositions were prepared by mixing thymol and 25 mM of mGN at various molar ratios, and their effects on GLP-1 secretion were examined. The experiment was carried out in the same manner as in Experiment 1-3 except that test samples shown in Table 3 were used. The number of samples (N) for each test sample was 3. The judgement of the synergistic effect was done in the same manner as in Experiment 2. The obtained results were shown in Table 3.

[Table 3]

| Test No. | Test Sample | | Independent Use | Combination | Molar Ratio (mGN: Thymol) | Synergistic Effect |
|---|---|---|---|---|---|---|
| Control | No Addition | | 100 | - | - | - |
| 3-1 | mGN | 25 mM (2% by mass) | 521 | 985 | 200:1 | ◎ |
| | Thymol | 0.125 mM (0.002% by mass) | 65 | | | |
| 3-2 | mGN | 25 mM (2% by mass) | 521 | 655 | 1,000:1 | ○ |
| | Thymol | 0.025 mM (0.0004% by mass) | 61 | | | |
| 3-3 | mGN | 25 mM (2% by mass) | 521 | 617 | 3,731:1 | ○ |
| | Thymol | 0.0067 mM (0.0001% by mass) | 34 | | | |

As shown in Table 3, thymol alone showed no GLP-1 secretion promoting effect at the concentrations in the range of 0.0067 mM to 0.125 mM. Rather, the decreased GLP-1 secretion was observed compared to Control in this range. In contrast, when 0.0067 mM to 0.125 mM of thymol was used in combination with mGN, surprisingly, GLP-1 secretion was remarkably promoted and furthermore the amount of GLP-1 secretion was greater than that when mGN was used alone. As shown in Table 3, in all of Tests Nos. 3-1 to 3-3, in which the formulation ratio of mGN and thymol in terms of the molar ratio was in the range of 3,731:1 to 200:1, the synergistic effect by the combination of mGN and thymol was observed. Notably, the particularly remarkable synergistic effect was observed in Test No. 3-1, in which mGN and thymol was used at the molar ratio of 200:1.

Based on the results of Experiment 2 and Experiment 3, it was judged that, when the composition of the present invention comprises a glycosyl naringenin and thymol, a glycosyl naringenin and thymol may be comprised at any formulation ratio, but that, from the viewpoint of obtaining the synergistic GLP-1 secretion-promoting effect, it may be preferable that the composition of the present invention comprises a glycosyl naringenin and thymol at a molar ratio in the range of 4,000:1 to 1:10, more preferably in the range of 3,731:1 to 1:10, and further preferably in the range of 200:1 to 1:1. Because the particularly great synergistic effect was observed for Tests Nos. 2-2, 2-3, and 2-4, it was concluded that it may be further more preferable that the composition of the present invention comprises a glycosyl naringenin and thymol at the molar ratio of 100:1 to 1:1.

<Experiment 4: Ratio of mGN and Nootkatone and its Effect on the GLP-1 Secretion-Promoting Effect>

In Experiment 1-3, the particularly good GLP-1 secretion-promoting effect was observed for the combination of mGN and thymol and the combination of mGN and nootkatone. Herein, the GLP-1 secretion-promoting effect shown by the combination of mGN and nootkatone was studied in more detail. Compositions comprising mGN and nootkatone at various molar ratios were prepared, and their effects on GLP-1 secretion were examined. The experiment was carried out in the same manner as in Experiment 1-3 except that test samples shown in Table 4 were used. The number of samples (N) for each test sample was 3. The judgement of the synergistic effect was done in the same manner as in Experiment 2. The obtained results were shown in Table 4.

[Table 4]

| Test No. | Test Sample | | Independent Use | Combination | Molar Ratio (mGN:NK) | Synergistic Effect |
|---|---|---|---|---|---|---|
| Control | No Addition | | 100 | - | - | - |
| 4-1 | mGN | 0.025 mM (0.002% by mass) | 39 | 493 | 1:5 | ○ |
| | NK | 0.125 mM (0.003% by mass) | 350 | | | |

(continued)

| Test No. | Test Sample | | Independent Use | Combination | Molar Ratio (mGN:NK) | Synergistic Effect |
|---|---|---|---|---|---|---|
| Control | No Addition | | 100 | - | - | - |
| 4-2 | mGN | 0.25 mM (0.02% by mass) | 63 | 791 | 1:1 | ◎ |
| | NK | 0.25 mM (0.006% by mass) | 481 | | | |
| 4-3 | mGN | 2.5 mM (0.2% by mass) | 85 | 1,005 | 10:1 | ◎ |
| | NK | 0.25 mM (0.006% by mass) | 464 | | | |
| 4-4 | | mGN 12.5 mM (0.3% by mass) | 502 | 968 | 100:1 | ○ |
| | NK | 0.125 mM (0.003% by mass) | 350 | | | |
| 4-5 | | mGN 12.5 mM (0.3% by mass) | 502 | 559 | 333:1 | ○ |
| | NK | 0.0375 mM (0.0008% by mass) | 111 | | | |
| 4-6 | | mGN 12.5 mM (0.3% by mass) | 502 | 610 | 1,000:1 | ○ |
| | NK | 0.0125 mM (0.0003% by mass) | 70 | | | |
| *NK: Nootkatone | | | | | | |

As shown in Table 4, even when nootkatone was used alone, the GLP-1 secretion was promoted in a nootkatone concentration-dependent manner, but the more remarkable promotion of GLP-1 secretion was observed when nootkatone and mGN were used in combination. In detail, the synergistic effect was observed in all of Tests Nos. 4-1 to 4-6, in which the molar ratio of mGN and nootkatone was in the range of 1,000:1 to 1:5. In particular, it was revealed that the GLP-1 secretion-promoting effect was more effectively enhanced in Tests Nos. 4-2 to 4-3, in which the molar ratio of mGN and nootkatone was in the range of 10:1 to 1:1. Based on the above results, it was judged that, when the composition of the present invention comprises a glycosyl naringenin and nootkatone, it may be preferable to comprise glycosyl naringenin and nootkatone at a molar ratio in the range of 1,000:1 to 1:5 and more preferably at a molar ratio in the range of 10:1 to 1:1 from the viewpoint of obtaining the synergistic GLP-1 secretion-promoting effect.

<Experiment 5: Effect of Formulation Ratio of mGN and a 1:1 Mixture of Thymol and Nootkatone on GLP-1 Secretion-Promoting Effect>

In order to study the effect of combination of three components, i.e., mGN, thymol and nootkatone, compositions comprising mGN and a mixture that was prepared by mixing thymol and nootkatone at a molar ratio of 1:1 (hereinafter referred to as "1:1 mixture of thymol and nootkatone") at various molar ratio were prepared, and their effects on the GLP-1 secretion was examined. The experiment was carried out in the same manner as in Experiment 1-3 except that test samples shown in Table 5 were used. The number of samples (N) for each test sample was 3. The judgement of the synergistic effect was done in the same manner as in Experiment 2. The obtained results were shown in Table 5. In Table 5, a molar concentration of the 1:1 mixture of thymol and nootkatone expresses a sum of a molar concentration of thymol in the mixture and a molar concentration of nootkatone in the mixture. A molar ratio of mGN and the 1:1 mixture of thymol and nootkatone was calculated based on this sum of molar concentrations.

[Table 5]

| Test No. | Test Sample | | Independent Use | Combination | Molar Ratio (mGN:Mixture) | Synergistic Effect |
|---|---|---|---|---|---|---|
| Control | No Addition | | 100 | - | - | - |
| 5-1 | mGN | 0.025 mM (0.002% by mass) | 39 | 632 | 1:10 | ◎ |
| | | 1:1 mixture of Thymol and Nootkatone 0.25 mM (0.005% by mass) | 440 | | | |
| 5-2 | mGN | 25 mM (2% by mass) | 457 | 559 | 600:1 | ○ |
| | | 1:1 mixture of Thymol and Nootkatone 0.042 mM (0.0008% by mass) | 105 | | | |
| 5-3 | | mGN 25 mM (2% by mass) | 586 | 739 | 1,000:1 | ○ |
| | | 1:1 mixture of Thymol and Nootkatone 0.025 mM (0.0005% by mass) | 90 | | | |

As shown in Table 5, when the 1:1 mixture of thymol and nootkatone that was prepared by mixing thymol and nootkatone at a molar ratio of 1:1 was used alone at concentrations in the range of 0.025 mM to 0.042 mM, there was almost no change in the amount of GLP-1 secretion compared to No Addition (Control) and therein no GLP-1 secretion promoting effect was observed. In contrast, when mGN and the 1:1 mixture of thymol and nootkatone at concentrations in the range of 0.025 mM to 0.042 mM were used in combination, the GLP-1 secretion was remarkably promoted compared to when mGN or the 1:1 mixture of thymol and nootkatone was used alone. Meanwhile, when the 1:1 mixture of thymol and nootkatone was used at a concentration of 0.25 mM, the GLP-1 secretion-promoting effect was observed compared to No Addition (Control), and surprisingly the GLP-1 secretion was remarkably promoted only by adding mGN at a concentration of as small as 0.025 mM.

As shown in Table 5, the synergistic effect by combination of mGN and the 1:1 mixture of thymol and nootkatone was observed in all of Tests Nos. 5-1 to 5-3, in which the molar ratio of mGN and the 1:1 mixture of thymol and nootkatone was in the range of 1,000:1 to 1:10. In particular, the synergistic effect was more remarkable in Test No. 5-1, in which the molar ratio of mGN and thymol was 1:10.

<Experiment 6: Effect of Formulation Ratio of mGN and a 1:2 Mixture of Thymol and Nootkatone on GLP-1 Secretion-Promoting Effect>

Compositions comprising mGN and a mixture that was prepared by mixing thymol and nootkatone at a molar ratio of 1:2 (hereinafter referred to as "1:2 mixture of thymol and nootkatone") at various molar ratio were prepared, and their effects on the GLP-1 secretion was examined. The experiment was carried out in the same manner as in Experiment 1-3 except that test samples shown in Table 6 were used. The number of samples (N) for each test sample was 3. The judgement of the synergistic effect was done in the same manner as in Experiment 2. The obtained results were shown in Table 6.

[Table 6]

| Test No. | Test Sample | | Independent Use | Combination | Molar Ratio (mGN:Mixture) | Synergistic Effect |
|---|---|---|---|---|---|---|
| Control | No Addition | | 100 | - | - | - |
| 6-1 | mGN | 0.025 mM (0.002% by mass) | 53 | 748 | 1:10 | ○ |
| | | 1:2 mixture of Thymol and Nootkatone 0.25 mM (0.005% by mass) | 676 | | | |

(continued)

| Test No. | Test Sample | | Independent Use | Combination | Molar Ratio (mGN:Mixture) | Synergistic Effect |
|---|---|---|---|---|---|---|
| Control | No Addition | | 100 | - | - | - |
| 6-2 | mGN | 25 mM (2% by mass) | 457 | 660 | 600:1 | ◎ |
| | | 1:2 mixture of Thymol and Nootkatone 0.042 mM (0.0008% by mass) | 110 | | | |
| 6-3 | mGN | 25 mM (2% by mass) | 457 | 608 | 1,000:1 | ◎ |
| | | 1:2 mixture of Thymol and Nootkatone 0.025 mM (0.0005% by mass) | 85 | | | |

As shown in Table 6, when the 1:2 mixture of thymol and nootkatone that was prepared by mixing thymol and nootkatone at a molar ratio of 1:2 was used at a concentration of 0.25 mM, the GLP-1 secretion-promoting effect was observed compared to No Addition (Control). When mGN was used in combination, the GLP-1 secretion was further promoted. When the 1:2 mixture of thymol and nootkatone was used alone at concentrations in the range of 0.025 mM to 0.042 mM, no GLP-1 secretion-promoting effect was observed. However, when mGN and the 1:2 mixture of thymol and nootkatone at concentrations in the range of 0.025 mM to 0.042 mM were used in combination, surprisingly, the GLP-1 secretion was remarkably promoted.

As shown in Table 6, the synergistic effect by combination of mGN and the 1:2 mixture of thymol and nootkatone was observed in all of Tests Nos. 6-1 to 6-3, in which the molar ratio of mGN and the 1:2 mixture of thymol and nootkatone was in the range of 1,000:1 to 1:10. In particular, the synergistic effect was more prominent in Tests Nos. 6-2 and 6-3, in which the molar ratio of mGN and the 1:2 mixture of thymol and nootkatone was in the range of 1,000:1 to 600:1.

Based on the results of Experiment 5 and Experiment 6, it was judged that, when the composition of the present invention comprises a glycosyl naringenin together with thymol and nootkatone, thymol and nootkatone may be comprised at any formulation ratio, but that, from the viewpoint of obtaining the synergistic GLP-1 secretion-promoting effect, it may be preferable that the composition of the present invention comprises a glycosyl naringenin and a mixture of thymol and nootkatone at a molar ratio in the range of 1,000:1 to 1:10.

The present invention is explained in further detail below based on examples. However, the scope of the present invention should not be limited to these examples.

**Example 1**

<Lemon-Flavored Beverage>

The 3"-α-monoglucosyl naringin composition obtained in Experiment 1-1 in an amount of 0.015 parts by mass, 30 parts by mass of reduced starch syrup, 30 parts by mass of granulated sugar, 3 parts by mass of honey, 0.07 parts by mass of acesulfame potassium, 0.03 parts by mass of sucralose, 0.3 parts by mass of vitamin C, 3 parts by mass of citric acid, 1 part by mass of sodium citrate, 1 part by mass of lemon flavor, 1 part by mass of yuzu flavor, 0.00003 parts by mass of thymol, and 0.001 parts by mass of nootkatone were added together and thoroughly stirred, wherein water was further added to make a total mass of 1,000 parts by mass. The mixture was then sterilized at 90°C for 30 seconds. The thus obtained lemon-flavored beverage had a good sharp aftertaste of sweetness, and had an initial fresh feeling and a bittersweet peel feeling. When the obtained lemon-flavored beverage was stored at 40°C for 4 weeks, the lemon flavor was well maintained even after storage and the generation of undesirable deterioration odor was also suppressed. This product can be advantageously used as a beverage for promoting GLP-1 secretion and inhibiting elevation of blood glucose level.

**Example 2**

<Carbonated Beverage>

Twenty (20) parts by mass of high fructose corn syrup, 0.1 parts by mass of acesulfame potassium, 0.02 parts by mass of sucralose, 2 parts by mass of citric acid, 0.5 parts by mass of sodium citrate, 0.015 parts by mass of the 3"-α-monoglucosyl naringin composition obtained in Experiment 1-1 were mixed with 197 parts by mass of water, and the mixture was stirred well until dissolved. To the mixture, an appropriate amount of lemon flavor, 0.0001 parts by mass of thymol, and 0.0001 parts by mass of nootkatone were further added. After stirring well, 750 parts by mass of carbonated water was added to obtain a carbonated beverage. The obtained carbonated beverage was imparted with an appropriate bitterness, had a good sharp aftertaste of sweetness, was refreshing to drink, and had well maintained lemon flavor. This product can be advantageously used as a beverage for promoting GLP-1 secretion and inhibiting elevation of blood glucose level.

**Example 3**

<Fruit Juice Beverage>

Hundred (100) parts by mass of high fructose corn syrup, 22 parts by mass of concentrated orange juice (1/5 concentrated fruit juice), 8 parts by mass of citric acid, 1 part by mass of vitamin C, 0.04 parts by mass of stevia, 0.03 parts by mass of the 3"-α-monoglucosyl naringin composition obtained in Experiment 1-1, 1 part by mass of orange flavor, 0.002 parts by mass of nootkatone, and 0.00001 parts by mass of thymol were mixed. Waster was further added to the mixture to make a total mass of 1,000 parts by mass to obtain a fruit juice beverage. The obtained fruit juice beverage had increased orange juice feeling. Because it contains nootkatone and thymol, the obtained fruit juice beverage had a further enhanced fruit juice feeling, had a good aroma, and was added with grapefruit-like flavor and bitterness, making it a fruit juice beverage with desirable characteristics. Furthermore, the aftertaste caused by high intensity sweetener was also reduced, resulting in a refreshing fruit juice beverage. This product can be advantageously used as a beverage for promoting GLP-1 secretion and inhibiting elevation of blood glucose level.

**Example 4**

<Shochu Highball (Chu Hai)>

The 3"-α-monoglucosyl naringin composition prepared in Experiment 1-1 in an amount of 0.05 parts by mass, 0.1 parts by mass of acesulfame potassium, 0.02 parts by mass of sucralose, 4 parts by mass of citric acid, and 1 part by mass of sodium citrate were mixed with 133 parts by mass of vodka with an alcohol concentration of 50% (v/v) (THE NIKKA WHISKY DISTILLING CO. LTD., Wilkinson Vodka 50°), and the mixture was stirred well until dissolved. To the mixture, an appropriate amount of lemon flavor, 0.0001 parts by mass of thymol, and 0.0001 parts by mass of nootkatone were further added. After stirring well, carbonated water was added to make a total mass of 1,000 parts by mass obtain a shochu highball. Although the obtained shochu highball has an alcohol concentration of 6%, it has an enhanced alcohol feeling with a fresh lemon flavor, clean and refreshing sweetness without unpleasant tastes, being an easy-to-drink shochu highball.

**Example 5**

<Beer-taste Beverage>

The 3"-α-monoglucosyl naringin composition prepared in Experiment 1-1 in an amount of 0.05 parts by mass, 2 parts by mass of α, α-trehalose, 2 parts by mass of malt extract, 0.02 parts by mass (as iso-α acid) of hop extract, 0.00005 parts by mass of thymol, and 0.001 parts by mass of nootkatone were dissolved in purified water and a total mass was made 1,000 parts by mass. The mixture was boiled for 1 hour. After cooling, the evaporated water was replenished, and then an appropriate amount of beer flavor was added. The mixture was clarified by diatomaceous earth filtration and filter filtration.

The thus obtained beer-taste beverage had favorable color tone, flavor, body feel, sharp bitterness, and feeling down the throat.

**Example 6**

<Tea Beverage>

To 25 g of green tea leaves, 800 g of deionized water at 70°C was added, and the tea components were extracted for 6 minutes. Then, the tea leaves were removed by filtration to obtain 700 mL of an extract. The obtained extract was diluted 3 times with deionized water. Therein, L-ascorbic acid was added at a concentration of 50 ppm, and then the pH was adjusted to 6.2 with sodium hydrogen carbonate. Next, the 3"-α-monoglucosyl naringin composition

prepared in Experiment 1-1 and thymol were added at a concentration of 10 ppm and 0.003 ppm, respectively. The mixture was dispensed in 350 ml portions into heat-resistant glass containers. After sealing the containers, they were sterilized at 121°C for 10 minutes to obtain a tea beverage.

The thus obtained green tea beverage was a beverage imparted with an appropriate bitterness and having an excellent aroma of tea. This product can be advantageously used as a beverage for promoting GLP-1 secretion and inhibiting elevation of blood glucose level.

**Example 7**

<Black Tea Beverage>

In accordance with the conventional method, a black tea extract was obtained by extracting black tea leaves with hot water. Three hundred twenty (320) parts by mass of this extract, 70 parts by mass of sugar, 0.015 parts by mass of the 3"-α-monoglucosyl naringin composition prepared in Experiment 1-1, and as flavoring agents, 0.1 part by mass of bergamot flavor, 0.00002 parts by mass of thymol, and 0.00026 parts by mass of nootkatone were mixed. The obtained mixture was diluted with deionized water and the pH was adjusted to 5.8 with baking soda to obtain a black tea in an amount of 1,000 parts by mass. The obtained black tea beverage was dispensed in 350 ml portions into cans and was retort sterilized at 120°C for 4 minutes. The thus obtained black tea beverage was a beverage imparted with an appropriate bitterness and astringency and having a black tea flavor and fruit juice feeling. This product can be advantageously used as a beverage for promoting GLP-1 secretion and inhibiting elevation of blood glucose level.

**Example 8**

<Coffee>

Hundreds (100) parts by mass of Arabica coffee beans roasted to have an L-value (the brightness of coffee beans measured with a color difference meter) of 22 were pulverized and extracted with hot water. The coffee bean extract was adjusted to Brix2, and to 500 parts by mass of this extract, 200 parts by mass of water, the 3"-α-monoglucosyl naringin composition prepared in Experiment 1-1, thymol and nootkatone were added at a concentration of 10 ppm, 0.011 ppm and 0.14 ppm, respectively. After adjusting pH, it was filled into cans and retort sterilized to obtain a canned coffee. The obtained canned coffee was an easy-to-drink canned coffee with the harmonized bitterness of coffee and having suppressed unpleasant taste and off-flavors. This product can be advantageously used as a beverage for promoting GLP-1 secretion and inhibiting elevation of blood glucose level.

**Example 9**

<Jelly>

Two hundred (200) parts by mass of sugar, 2.5 parts by mass of κ-carrageenan, 2.5 parts by mass of locust bean gum, 2.5 parts by mass of sodium citrate, 1 part by mass of potassium chloride, 0.2 parts by mass of acesulfame potassium were well mixed and added to 650 parts by mass of water in a pot, taking care to avoid formation of lumps. Therein, 0.01 parts by mass of the 3"-α-monoglucosyl naringin composition prepared in Experiment 1-1 were added. The mixture was boiled to evaporate water until the total mass of the mixture reached 800 parts by mass. Thereafter, the mixture was cooled to 70°C and then 35 parts by mass of concentrated orange juice (1/5 concentrated juice), an appropriate amount of orange flavor, 0.00003 parts by mass of thymol, 0.001 parts by mass of nootkatone were added. The pH of the mixture was adjusted to the range of 3.2 to 4.0 with citric acid 50% aqueous solution. The thus obtained solution was filled into a jelly cup and left to stand in a refrigerator until it got solidified, to obtain an orange jelly. The obtained orange jelly had an enhanced feeling of orange juice and feeling of freshness, and it was difficult to perceive the unpleasant taste derived from sweeteners. This product can be advantageously used as a food for promoting GLP-1 secretion and inhibiting elevation of blood glucose level.

**Example 10**

<Frozen Confectionery>

In 300 parts by mass of grapefruit juice and 350 parts by mass of water, 30 parts by mass of sugar, 20 parts by mass of α, α-trehalose, 0.1 parts by mass of grapefruit flavor, 0.015 parts by mass of the 3"-α-monoglucosyl naringin composition prepared in Experiment 1-1, 0.000005 parts by mass of thymol, and 0.001 parts by mass of nootkatone

were completely dissolved. The mixture was stirred with a sorbeture (a sherbet maker) while being frozen, to produce a grapefruit-flavored sherbet. The obtained sherbet was filled in a plastic cup and stored in a freezer at -20°C. On the other hand, a control frozen confectionary was also prepared in the same manner as above except that the 3''-α-monoglucosyl naringin composition was not blended. The frozen confectionary according to one embodiment of the present invention had a refreshing and low sweetness since it had a low solid and sugar content, and furthermore it had a better feeling of grapefruit juice and flavor compared to the control.

**Example 11**

<Gummy>

In accordance with a conventional method, 170 parts by mass of sugar, 250 parts by mass of a maltose syrup (Product Name "MALTRUP", manufactured by Hayashibara Co., Ltd.), 24 parts by mass of gelatin, 0.1 parts by mass of the 3''-α-monoglucosyl naringin composition prepared in Experiment 1-1, 48 parts by mass of water, 12 parts by mass of citric acid 50% aqueous solution, 0.6 parts by mass of grapefruit flavor, an appropriate amount of a coloring agent, 0.0001 parts by mass of thymol, 0.0001 parts by mass of nootkatone were mixed. The mixture was filled in a starch mold, dried for 1 day, and molded to obtain a gummy.

Since the composition of the present invention has excellent water solubility, the thus obtained gummy was transparent gummy with a slight bitterness and a fresh feeling of grapefruit.

**Example 12**

<Hard Candy>

Two hundred forty (240) parts by mass of high fructose corn syrup, 220 parts by mass of a maltose syrup (Product Name "MALTRUP", manufactured by Hayashibara Co., Ltd.), 80 parts by mass of water were mixed and boiled to 150°C. After cooling to 120°C, 0.01 parts by mass of the 3''-α-monoglucosyl naringin composition prepared in Experiment 1-1, 0.8 parts by mass of peppermint oil, 1.1 parts by mass of menthol, 0.005 parts by mass of thymol, 0.00025 parts by mass of nootkatone, and an appropriate amount of a coloring agent were added. The mixture was then molded to produce a candy. The obtained hard candy had an enhanced mint flavor and an increased refreshing feeling. Since the hard candy contains the 3''-α-monoglucosyl naringin composition and thymol, it has an antibacterial effect and an effect of reducing roughness, inflammation, pain, etc., in the oral cavity and the throat. The hard candy can be used for purpose of maintaining and promoting oral health.

**Example 13**

<Nutrition Drink>

An amino acid mixture was prepared by mixing 4 parts by mass of isoleucine, 6 parts by mass of leucine, 8 parts by mass of lysine, 8 parts by mass of phenylalanine, 1 part by mass of tyrosine, 12 parts by mass of tryptophan, 8 parts by mass of valine, 1 part by mass of aspartic acid, 1 part by mass of serine,8 parts by mass of aminoacetic acid, 8 parts by mass of alanine, 2 parts by mass of histidine, 8 parts by mass of arginine, 2 parts by mass of threonine and 1 part by mass of methionine. The obtained mixture was dissolved in water to obtain 2% solution of the mixture. To 50 parts by mass of this solution, 0.5 parts by mass of ascorbic acid, 0.01 parts by mass of nicotinamide, 0.01 parts by mass of vitamin B1, 0.01 parts by mass of vitamin B2, 0.01 parts by mass of vitamin B6, 1.8 parts by mass of concentrated grapefruit juice (1/5 concentrated fruit juice), 0.01 parts by mass of the 3''-α-monoglucosyl naringin composition prepared in Experiment 1-1, 0.0003 parts by mass of thymol, and 0.003 parts by mass of nootkatone were added. Therein, water was further added to make the total mass of 100 parts by mass, to obtain a nutrition drink. This product can be advantageously used for promoting GLP-1 secretion and inhibiting elevation of blood glucose level, and is a nutrition drink with good taste, improved palatability, and the reduced unpleasant tastes of the amino acids, wherein no browning or generation of off-flavor is observed even after storage for a long period of time.

**Example 14**

<Capsule>

Ten (10) parts by mass of calcium acetate monohydrate, 50 parts by mass of magnesium L-lactate trihydrate, 60 parts by mass of maltose, 30 parts by mass of the 3''-α-monoglucosyl naringin composition prepared in Experiment

1-1, 0.1 parts by mass of thymol, and 0.06 parts by mass of nootkatone were uniformly mixed, and the obtained mixture was granulated by a granulator. The obtained granules were filled in a gelatin capsule in accordance with a conventional method, to obtain a supplement in a capsule form with 150 mg of the granules per capsule. This product can be advantageously used as an oral composition for promoting GLP-1 secretion, inhibiting elevation of blood glucose level, and promoting health.

**Example 15**

<Tablets>

In accordance with a conventional method, 5 parts by mass of the 3"-α-monoglucosyl naringin composition prepared in Experiment 1-1, 68 parts by mass of maltose, 15 parts by mass of α, α-trehalose, 5 parts by mass of pullulan-containing powder, 0.12 parts by mass of thymol, and 0.1 parts by mass of grapefruit flavor were uniformly mixed, and the obtained mixture was tableted to obtain a tablet (200 mg per tablet).

This product not only has an appropriate strength but also has good flavor and taste, being advantageous as a tablet for promoting GLP-1 secretion and inhibiting elevation of blood glucose level. Furthermore, since this product contains thymol, it is also advantageous as an oral composition for promoting health, such as for preventing and/or improving sensitivity to cold by improving and maintaining blood flow.

**Example 16**

<Water-Soluble Flavoring>

Lemon oil and 65 w/w% water-containing ethanol were mixed. After stirring the mixture, the mixture was left at low temperature to allow a phase separation to a water layer (essence layer) containing water-soluble flavor components in water-containing ethanol and an oil layer containing water-insoluble components. The water layer was collected to obtain a lemon essence. To 98 parts by mass of the obtained lemon essence, 2 parts by mass of the 3"-α-monoglucosyl naringin composition prepared in Experiment 1-1, 0.01 parts by mass of thymol, and 0.05 parts by mass of nootkatone were mixed, to obtain a water-soluble lemon flavor. The obtained lemon flavor had reduced deterioration odor due to long-term storage and retained good aroma and flavor. Since the composition of the present invention has excellent water solubility, the obtained lemon flavor can be used as a water-soluble and highly stable liquid flavoring. This product can be added to foods or beverages, usually, in an amount in the range of 0.01 to 1% to enhance development and persistence of flavor. In addition, this product can be added to foods or beverages to obtain an oral composition advantageously used for promoting GLP-1 secretion, inhibiting elevation of blood glucose level, and promoting health.

**Example 17**

<Powdered Flavoring>

To 100 g of water, 5 g of sucrose fatty acid ester with an HLB value of 15, 2 g of the 3"-α-monoglucosyl naringin composition prepared in Experiment 1-1 were added and dissolved, followed by heat sterilization at 85 to 90°C for 15 minutes. After cooling the obtained solution to about 40°C, 10 g of lemon oil, 0.001 g of thymol and 0.5 g of nootkatone were added and mixed while stirring using a homomixer, to obtain an emulsified flavoring. This product can be further dried by a method such as spray-drying to easily obtain a powered flavoring. The obtained powdered flavoring had no deterioration odor due to long-term storage and retained good aroma and flavor.

This product can be added to foods or beverages, usually, in an amount in the range of 0.1 to 3% to obtain an oral composition advantageously used for promoting GLP-1 secretion, inhibiting elevation of blood glucose level, and promoting health.

**Industrial Applicability**

As described above, because the composition of the present invention has the good GLP-1 secretion promoting effect, it may be advantageously used for preventing or improving obesity, diabetes, and postprandial hyperglycemia, as well as inhibiting elevation of blood glucose level, inhibiting glucagon secretion, inhibiting gastric excretion and gastric acid secretion, regulating appetite, etc., to which promoting GLP-1 secretion is be effective. It may be provided in the form of foods, supplements, pharmaceuticals, quasi-drugs, flavoring agents, and the like.

## Claims

1. A composition for promoting GLP-1 secretion, comprising component (A) and component (B) as active ingredients, wherein said component (A) is a glycosyl naringenin and said component (B) is one or more selected from a group consisting of α-pinene, β-pinene, sabinene, camphene, valencene, β-caryophyllene, β-ionone, methyl anthranilate, methyl N-methylanthranilate, thymol, and nootkatone.

2. The composition for promoting GLP-1 secretion according to claim 1, wherein said component (A) is 3"-α-monoglucosyl naringin.

3. The composition for promoting GLP-1 secretion according to claim 1 or 2, wherein said component (B) is thymol and/or nootkatone.

4. The composition for promoting GLP-1 secretion according to any one of claims 1 to 3, comprising said component (A) and said component (B) at a molar ratio of 10,000:1 to 1:10.

5. The composition for promoting GLP-1 secretion according to any one of claims 1 to 4, comprising said component (A) in an amount of 0.0001% by mass or more.

6. The composition for promoting GLP-1 secretion according to any one of claims 1 to 5, which is for use in at least one of the followings: prevention or improvement of obesity, diabetes, and postprandial hyperglycemia; suppression of elevation of blood glucose level; inhibition of glucagon secretion; inhibition of gastric excretion and gastric acid secretion; suppression of appetite; suppression of food intake; and induction of satiety.

7. A composition, comprising component (A) and component (B), wherein said component (A) is a glycosyl naringenin and said component (B) is one or more selected from a group consisting of α-pinene, β-pinene, sabinene, camphene, valencene, β-caryophyllene, β-ionone, methyl anthranilate, methyl N-methylanthranilate, thymol, and nootkatone.

8. The composition according to claim 7, comprising said component (A) and said component (B) at a molar ratio of 10,000:1 to 1:10.

9. The composition according to claim 7 or 8, wherein said component (A) is 3"-α-monoglucosyl naringin.

10. The composition according to any one of claims 7 to 9, wherein said component (B) is thymol and/or nootkatone.

11. The composition according to any one of claims 7 to 10, comprising said component (A) in an amount of 0.0001% by mass or more.

12. A food or beverage, comprising the composition according to any one of claims 1 to 11.

13. A supplement, comprising the composition according to any one of claims 1 to 11.

14. A flavoring agent, comprising the composition according to any one of claims 1 to 11.

15. A pharmaceutical or quasi-drug, comprising the composition according to any one of claims 1 to 11.

16. A feed, comprising the composition according to any one of claims 1 to 11.

17. The food or beverage according to claim 12, which is in the form of liquid, fluid, gel, semi-solid, solid or powder.

18. The supplement according to claim 13, which is in the form of liquid, gel, paste, tablet, round tablet, capsule, powder, granule, fine granule, or troche.

19. The flavoring agent according to claim 14, which is in the form of a water-soluble flavoring, oil-soluble flavoring, emulsified flavoring, or powdered flavoring.

20. The pharmaceutical or quasi-drug according to claim 15, which is in the form of liquid, fluid, gel, semi-solid, solid, tablet, round tablet, capsule, powder, granule, fine granule or troche.

**21.** The feed according to claim 16, which is in the form of liquid, powder, pellet, flake, or mash.

**INTERNATIONAL SEARCH REPORT**

International application No. **PCT/JP2022/015872**

**A. CLASSIFICATION OF SUBJECT MATTER**

***A23L 33/10***(2016.01)i; ***A23F 3/16***(2006.01)i; ***A23F 5/24***(2006.01)i; ***A23G 3/36***(2006.01)i; ***A23G 9/32***(2006.01)i; ***A23L 2/52***(2006.01)i; ***A61K 9/06***(2006.01)i; ***A61K 9/08***(2006.01)i; ***A61K 9/107***(2006.01)i; ***A61K 9/16***(2006.01)i; ***A61K 9/20***(2006.01)i; ***A61K 9/48***(2006.01)i; ***A61K 31/05***(2006.01)i; ***A61K 31/122***(2006.01)i; ***A61K 31/7048***(2006.01)i; ***A61P 1/04***(2006.01)i; ***A61P 3/04***(2006.01)i; ***A61P 3/10***(2006.01)i; ***A61P 43/00***(2006.01)i; ***C12G 3/05***(2019.01)i

FI: A23L33/10; A23F3/16; A23F5/24; A23G3/36; A23G9/32; A23L2/00 F; A23L2/52; A61K9/06; A61K9/08; A61K9/107; A61K9/16; A61K9/20; A61K9/48; A61K31/05; A61K31/122; A61P1/04; A61P3/04; A61P3/10; A61P43/00 111; A61P43/00 121; C12G3/05; A61K31/7048

According to International Patent Classification (IPC) or to both national classification and IPC

**B. FIELDS SEARCHED**

Minimum documentation searched (classification system followed by classification symbols)

A23L33/10; A23F3/16; A23F5/24; A23G3/36; A23G9/32; A23L2/52; A61K9/06; A61K9/08; A61K9/107; A61K9/16; A61K9/20; A61K9/48; A61K31/05; A61K31/122; A61K31/7048; A61P1/04; A61P3/04; A61P3/10; A61P43/00; C12G3/05

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/039059 A1 (DELACON BIOTECHNIK GMBH) 27 February 2020 (2020-02-27) claims, p. 5, line 18 to p. 7, line 22, examples | 7-8, 10-11, 16, 21 |
| X | JP 2019-533009 A (GRACE BREEDING LTD) 14 November 2019 (2019-11-14) claims, paragraph [0034], examples | 7-8, 10-11, 15, 20 |
| A | US 2015/0343007 A1 (CONOPCO INC DBA UNILEVER) 03 December 2015 (2015-12-03) claims, examples | 1-21 |
| A | JP 2017-169527 A (TOYO SUGAR REFINING) 28 September 2017 (2017-09-28) paragraphs [0090]-[0093], example 4 | 1-21 |
| A | JP 2013-173713 A (UNIV OF TOKYO) 05 September 2013 (2013-09-05) claims, paragraph [0038], example 3 | 1-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 May 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/015872**

**C. DOCUMENTS CONSIDERED TO BE RELEVANT**

| Category* | Citation of document, with indication, where appropriate, of the relevant passages | Relevant to claim No. |
|---|---|---|
| A | WO 2020/254663 A2 (SOCIETE DES PRODUITS NESTLE S.A.) 24 December 2020 (2020-12-24)<br>claims, paragraphs [0137]-[0141] | 1-21 |
| A | JP 2007-063241 A (KAO CORP) 15 March 2007 (2007-03-15)<br>claims, examples | 1-21 |

Form PCT/ISA/210 (second sheet) (January 2015)

**INTERNATIONAL SEARCH REPORT**
**Information on patent family members**

International application No.
**PCT/JP2022/015872**

| Patent document cited in search report | Publication date (day/month/year) | Patent family member(s) | Publication date (day/month/year) |
|---|---|---|---|
| WO 2020/039059 A1 | 27 February 2020 | US 2021/0236577 A1<br>claims, paragraphs [0031]-[0038], examples<br>EP 3840588 A1<br>CN 113056204 A | |
| JP 2019-533009 A | 14 November 2019 | US 2019/0247449 A1<br>claims, paragraph [0066], examples<br>WO 2018/051344 A1<br>EP 3512342 A1<br>CN 110234228 A | |
| US 2015/0343007 A1 | 03 December 2015 | WO 2014/090569 A1<br>claims, examples<br>CN 104837370 A | |
| JP 2017-169527 A | 28 September 2017 | (Family: none) | |
| JP 2013-173713 A | 05 September 2013 | (Family: none) | |
| WO 2020/254663 A2 | 24 December 2020 | EP 3986386 A2<br>claims, paragraphs [0137]-[0141]<br>CN 113905727 A | |
| JP 2007-063241 A | 15 March 2007 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016138070 A
- WO 2017159725 A
- JP H0413691 B
- JP 2007284393 A
- JP 2002199896 A


### Non-patent literature cited in the description


- Incretin. *Gekkan Tonyobyo (DIABETES),* December 2009
- *Biochemical and Biophysical Research Communications,* 2009, vol. 380, 44-49
- *PLoS ONE,* 2013, vol. 8 (8), e70933
- *Journal of Endocrinology,* 2013, vol. 219, 59-68
- *PLoS ONE,* 2013, vol. 8 (11), e81119
- *Scientific Reports,* 2017, vol. 7 (1), 1-11